(19) **Europäisches Patentamt / European Patent Office / Office européen des brevets**

(11) **EP 4 296 357 A1**

(12) # EUROPEAN PATENT APPLICATION
published in accordance with Art. 153(4) EPC

(43) Date of publication:
**27.12.2023 Bulletin 2023/52**

(21) Application number: **22756287.3**

(22) Date of filing: **18.02.2022**

(51) International Patent Classification (IPC):
*C12N 15/13* (2006.01)  *A61K 39/395* (2006.01)
*A61P 13/12* (2006.01)  *A61P 19/02* (2006.01)
*A61P 29/00* (2006.01)  *A61P 37/02* (2006.01)
*A61P 37/06* (2006.01)  *A61P 43/00* (2006.01)
*C07K 16/40* (2006.01)  *C12N 1/15* (2006.01)
*C12N 1/19* (2006.01)  *C12N 1/21* (2006.01)
*C12N 5/10* (2006.01)

(52) Cooperative Patent Classification (CPC):
**A61K 39/395; A61P 13/12; A61P 19/02;
A61P 29/00; A61P 37/02; A61P 37/06;
A61P 43/00; C07K 16/00; C07K 16/40; C12N 5/10;
C12N 15/74; C12N 15/80; C12N 15/81**

(86) International application number:
**PCT/JP2022/006528**

(87) International publication number:
**WO 2022/176970 (25.08.2022 Gazette 2022/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.02.2021 JP 2021024642**

(71) Applicants:
- **Mitsubishi Tanabe Pharma Corporation
  Osaka-shi,
  Osaka 541-8505 (JP)**
- **Pharma Foods International Co., Ltd.
  Kyoto 615-8245 (JP)**

(72) Inventors:
- **MIYAMOTO, Yuya
  Osaka-shi, Osaka 541-8505 (JP)**

- **WADA, Koichi
  Osaka-shi, Osaka 541-8505 (JP)**
- **IMURA, Yuichi
  Osaka-shi, Osaka 541-8505 (JP)**
- **SAITO, Kenji
  Kyoto-shi, Kyoto 615-8245 (JP)**
- **SAKATA, Tomoko
  Kyoto-shi, Kyoto 615-8245 (JP)**
- **SHIGEMITSU, Takanari
  Kyoto-shi, Kyoto 615-8245 (JP)**

(74) Representative: **J A Kemp LLP
80 Turnmill Street
London EC1M 5QU (GB)**

Remarks:
The complete document including Reference
Table(s) and the Sequence Listing(s) can be
downloaded from the EPO website

(54) **NOVEL ANTI-PAD4 ANTIBODY**

(57)    Provided is an anti-PAD4 antibody or an antibody fragment thereof, wherein HCDR1 comprises an amino acid sequence of SEQ ID NO: 1, HCDR2 comprises an amino acid sequence of SEQ ID NO: 2, HCDR3 comprises an amino acid sequence of SEQ ID NO: 3, LCDR1 comprises an amino acid sequence of SEQ ID NO: 4, LCDR2 comprises an amino acid sequence of SEQ ID NO: 5, and LCDR3 comprises an amino acid sequence of SEQ ID NO: 6.

Fig. 2

**Description**

TECHNICAL FIELD

The present invention relates to a novel anti-PAD4 antibody.

BACKGROUND ART

PAD4 (Peptidylarginine deiminase 4) is known as an enzyme involved in citrullination of arginine in proteins. The citrullination is a reaction in which arginine, which is the most basic among amino acids that make up proteins, is converted to neutral citrulline, and thus, is important for structures and reactions of proteins.

Citrullination has been reported to be associated with rheumatoid arthritis (RA). For example, in RA, vimentin, collagen, fibrin, and the like are present as antigens in the synovium, and thus, a detection kit for anti-cyclic citrullinated peptide antibody (anti-CCP antibody), which is an antibody against these, is marketed as a diagnostic agent for RA.

There are also several reports on PAD4 and RA. For example, Non-Patent Document 1 reports that there is a correlation between the onset of RA and the single nucleotide polymorphism of the PAD4 gene. Further, Non-Patent Document 2 reports that an anti-PAD4 antibody has been used for diagnosing RA. Further, Patent Document 1 describes an attempt to suppress RA by administering a mixture of four types of anti-PAD4 antibodies to mice (see Example 2 of Patent Document 1). Further, Patent Document 2 describes an anti-PAD4 antibody that is superior in its affinity to PAD4 and its ability to inhibit citrullination activity.

As reports on PAD4 and systemic lupus erythematosus (SLE), there are a report that nephritis is suppressed in SLE model (imikimod induction model) using PAD4 knockout mouse as compared to wild strains and a report that nephritis in SLE model (MRL/lpr model) mice is suppressed by administration of small molecule PAD inhibiting agents (Cl-Amidine and BB-Cl-Amidine) (Non-Patent Documents 3, 4, 5). Further, there are a report that SLE-like symptoms developed in PAD4 knockout MRL/lpr mice similar to wild strains and a report that administration of a small molecule inhibiting agent (Cl-Amidine) to a nephritis model (anti-GBM antibody-induced model and SLE patient serum-transferred mice) failed to suppress the onset of nephritis (Non-Patent Document 6).

From the relationship between such diseases and PAD4, an anti-PAD4 antibody with further superior binding affinity and stability is desired.

PRIOR ART DOCUMENTS

[Patent Documents]

[Patent Document 1] WO 2012/026309

[Patent Document 2] WO 2016/143753

[Non-Patent Documents]

[Non-Patent Document 1] "Functional haplotypes of PADI4, encoding citrullinating enzyme peptidylarginine deiminase 4, are associated with rheumatoid arthritis," Suzuki et al., Nat Genet., 2003 Aug; 34 (4): 395-402.

[Non-Patent Document 2] "Two novel sandwich ELISAs identify PAD4 levels and PAD4 autoantibodies in patients with rheumatoid arthritis," Ishigami et al., Mod Rheumatol., 2013 Jul; 23 (4): 794-803.

[Non-Patent Document 3] "Peptidylarginine deiminases 2 and 4 modulate innate and adaptive immune responses in TLR-7-dependent lupus," Yudong et al., JCI Insight., 2018 Dec 6; 3 (23): e124729.

[Non-Patent Document 4] "Peptidylarginine Deiminase 4 Promotes the Renal Infiltration of Neutrophils and Exacerbates the TLR7 Agonist-Induced Lupus Mice," Hanata et al., Front Immunol., 2020 Jun 23; 11:1095.

[Non-Patent Document 5] "Peptidylarginine deiminase inhibition disrupts NET formation and protects against kidney, skin and vascular disease in lupus-prone MRL/lpr mice," Knight et al., Ann Rheum Dis., 2015 Dec; 74 (12): 2199-2206.

[Non-Patent Document 6] "Lupus and proliferative nephritis are PAD4 independent in murine models," Gordon et al., JCI Insight., 2017 May 18; 2 (10): e92926.

SUMMARY OF THE INVENTION

PROBLEMS TO BE SOLVED BY THE INVENTION

The present invention is accomplished in view of the above-described situation, and is intended to provide an anti-PAD4 antibody having excellent properties, or to provide an excellent preventive or therapeutic method for RA or arthritis, systemic lupus erythematosus, lupus nephritis, or graft-versus-host disease, or the like.

MEANS FOR SOLVING THE PROBLEMS

The present inventors have conducted extensive studies to solve the above-described problem. As a result, the present inventors have found that an anti-PAD4 antibody having a specific complementarity determining region (CDR) sequence has excellent binding property and storage stability. Further, the present inventors have found that an anti-PAD4 antibody having a specific framework region (FR) sequence has an excellent chemical stability, and thus have accomplished the present invention.

That is, a summary of the present invention is as follows.

[1] An anti-PAD4 antibody or an antibody fragment thereof, wherein HCDR1 comprises an amino acid sequence of SEQ ID NO: 1, HCDR2 comprises an amino acid sequence of SEQ ID NO: 2, HCDR3 comprises an amino acid sequence of SEQ ID NO: 3, LCDR1 comprises an amino acid sequence of SEQ ID NO: 4, LCDR2 comprises an amino acid sequence of SEQ ID NO: 5, and LCDR3 comprises an amino acid sequence of SEQ ID NO: 6.

[2] The anti-PAD4 antibody or the antibody fragment thereof according to [1], wherein HCDR1 comprises the amino acid sequence of SEQ ID NO: 1, HCDR2 comprises any one of amino acid sequences of SEQ ID NOs: 7 - 10, HCDR3 comprises an amino acid sequence of SEQ ID NO: 11 or 12, LCDR1 comprises the amino acid sequence of SEQ ID NO: 4, LCDR2 comprises the amino acid sequence of SEQ ID NO: 5, and LCDR3 comprises any one of amino acid sequences of SEQ ID NOs: 13 - 15.

[3] The anti-PAD4 antibody or the antibody fragment thereof according to [1] or [2], wherein said anti-PAD4 antibody or the antibody fragment thereof is selected from the group consisting of:

(a-1) an anti-PAD4 antibody or an antibody fragment thereof, wherein HCDR1 comprises the amino acid sequence of SEQ ID NO: 1, HCDR2 comprises the amino acid sequence of SEQ ID NO: 7, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 13;

(b-1) an anti-PAD4 antibody or an antibody fragment thereof, wherein HCDR1 comprises the amino acid sequence of SEQ ID NO: 1, HCDR2 comprises the amino acid sequence of SEQ ID NO: 8, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 13;

(c-1) an anti-PAD4 antibody or an antibody fragment thereof, wherein HCDR1 comprises the amino acid sequence of SEQ ID NO: 1, HCDR2 comprises the amino acid sequence of SEQ ID NO: 9, HCDR3 comprises the amino acid sequence of SEQ ID NO: 12, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 14; and

(d-1) an anti-PAD4 antibody or an antibody fragment thereof, wherein HCDR1 comprises the amino acid sequence of SEQ ID NO: 1, HCDR2 comprises the amino acid sequence of SEQ ID NO: 10, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 15.

[4] The anti-PAD4 antibody or the antibody fragment thereof according to any one of [1] - [3], wherein said anti-PAD4 antibody or the antibody fragment thereof is selected from the group consisting of:

(a-2) an anti-PAD4 antibody or an antibody fragment thereof, wherein HCDR1 consists of the amino acid sequence of SEQ ID NO: 1, HCDR2 consists of the amino acid sequence of SEQ ID NO: 7, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 13;

(b-2) an anti-PAD4 antibody or an antibody fragment thereof, wherein HCDR1 consists of the amino acid sequence of SEQ ID NO: 1, HCDR2 consists of the amino acid sequence of SEQ ID NO: 8, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 13;

(c-2) an anti-PAD4 antibody or an antibody fragment thereof, wherein HCDR1 consists of the amino acid sequence of SEQ ID NO: 1, HCDR2 consists of the amino acid sequence of SEQ ID NO: 9, HCDR3 consists of the amino acid sequence of SEQ ID NO: 12, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 14; and

(d-2) an anti-PAD4 antibody or an antibody fragment thereof, wherein HCDR1 consists of the amino acid sequence of SEQ ID NO: 1, HCDR2 consists of the amino acid sequence of SEQ ID NO: 10, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 15.

[5] An anti-PAD4 antibody or an antibody fragment thereof, wherein said anti-PAD4 antibody or the antibody fragment thereof is selected from the group consisting of:

(a-3) an anti-PAD4 antibody or an antibody fragment thereof, wherein a heavy chain variable region comprises an amino acid sequence of amino acid numbers 1 - 120 of SEQ ID NO: 16, and a light chain variable region comprises an amino acid sequence of amino acid numbers 1 - 105 of SEQ ID NO: 17;

(b-3) an anti-PAD4 antibody or an antibody fragment thereof, wherein a heavy chain variable region comprises an amino acid sequence of amino acid numbers 1 - 120 of SEQ ID NO: 18, and a light chain variable region comprises an amino acid sequence of amino acid numbers 1 - 105 of SEQ ID NO: 19;

(c-3) an anti-PAD4 antibody or an antibody fragment thereof, wherein a heavy chain variable region comprises an amino acid sequence of amino acid numbers 1 - 120 of SEQ ID NO: 20, and a light chain variable region comprises an amino acid sequence of amino acid numbers 1 - 105 of SEQ ID NO: 21; and

(d-3) an anti-PAD4 antibody or an antibody fragment thereof, wherein a heavy chain variable region comprises an amino acid sequence of amino acid numbers 1 - 120 of SEQ ID NO: 22, and a light chain variable region comprises an amino acid sequence of amino acid numbers 1 - 105 of SEQ ID NO: 23.

[6] The anti-PAD4 antibody or the antibody fragment thereof according to [5], wherein said anti-PAD4 antibody or the antibody fragment thereof is selected from the group consisting of:

(a-4) an anti-PAD4 antibody or an antibody fragment thereof, wherein a heavy chain variable region consists of the amino acid sequence of amino acid numbers 1 - 120 of SEQ ID NO: 16, and a light chain variable region consists of the amino acid sequence of amino acid numbers 1 - 105 of SEQ ID NO: 17;

(b-4) an anti-PAD4 antibody or an antibody fragment thereof, wherein a heavy chain variable region consists of the amino acid sequence of amino acid numbers 1 - 120 of SEQ ID NO: 18, and a light chain variable region consists of the amino acid sequence of amino acid numbers 1 - 105 of SEQ ID NO: 19;

(c-4) an anti-PAD4 antibody or an antibody fragment thereof, wherein a heavy chain variable region consists of the amino acid sequence of amino acid numbers 1 - 120 of SEQ ID NO: 20, and a light chain variable region consists of the amino acid sequence of amino acid numbers 1 - 105 of SEQ ID NO: 21; and

(d-4) an anti-PAD4 antibody or an antibody fragment thereof, wherein a heavy chain variable region consists of the amino acid sequence of amino acid numbers 1 - 120 of SEQ ID NO: 22, and a light chain variable region consists of the amino acid sequence of amino acid numbers 1 - 105 of SEQ ID NO: 23.

[7] The anti-PAD4 antibody or the antibody fragment thereof according to [5] or [6], wherein said anti-PAD4 antibody or the antibody fragment thereof is selected from the group consisting:

(a-5) an anti-PAD4 antibody or an antibody fragment thereof, wherein a heavy chain consists of an amino acid sequence of SEQ ID NO: 16, and a light chain consists of an amino acid sequence of SEQ ID NO: 17;

(b-5) an anti-PAD4 antibody or an antibody fragment thereof, wherein a heavy chain consists of an amino acid sequence of SEQ ID NO: 18, and a light chain consists of an amino acid sequence of SEQ ID NO: 19;

(c-5) an anti-PAD4 antibody or an antibody fragment thereof, wherein a heavy chain consists of an amino acid sequence of SEQ ID NO: 20, and a light chain consists of an amino acid sequence of SEQ ID NO: 21; and

(d-5) an anti-PAD4 antibody or an antibody fragment thereof, wherein a heavy chain consists of an amino acid sequence of SEQ ID NO: 22, and a light chain consists of an amino acid sequence of SEQ ID NO: 23.

[8] The anti-PAD4 antibody or the antibody fragment thereof according to any one of [1] - [7], wherein an amino acid corresponding to the 84th amino acid of a heavy chain is serine.

[9] The anti-PAD4 antibody or the antibody fragment thereof according to any one of [1] - [8], wherein a KD value with respect to PAD4 is 100 pM or less.

[10] The anti-PAD4 antibody or the antibody fragment thereof according to any one of [1] - [9] wherein said anti-PAD4 antibody or the antibody fragment thereof has a binding amount of 90% or more when the anti-PAD4 antibody or the antibody fragment thereof is stored at 40 °C for one month as compared to a PAD4-binding amount of the anti-PAD4 antibody or the antibody fragment thereof before the storage.

[11] (e-1) An anti-PAD4 antibody or an antibody fragment thereof, wherein HCDR1 comprises an amino acid sequence of SEQ ID NO: 1, HCDR2 comprises an amino acid sequence of SEQ ID NO: 24, HCDR3 comprises an amino acid sequence of SEQ ID NO: 11, LCDR1 comprises an amino acid sequence of SEQ ID NO: 4, LCDR2 comprises an amino acid sequence of SEQ ID NO: 5, and LCDR3 comprises an amino acid sequence of SEQ ID NO: 25, wherein an amino acid corresponding to the 84th amino acid of a heavy chain is serine.

[12] (e-2) The anti-PAD4 antibody or the antibody fragment thereof according to [11], wherein HCDR1 consists of the amino acid sequence of SEQ ID NO: 1, HCDR2 consists of the amino acid sequence of SEQ ID NO: 24, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 25, wherein an amino acid corresponding to the 84th amino acid of a heavy chain is serine.

[13] (e-3) An anti-PAD4 antibody or an antibody fragment thereof, wherein a heavy chain variable region comprises an amino acid sequence of amino acid numbers 1 - 120 of SEQ ID NO: 28, and a light chain variable region comprises an amino acid sequence of amino acid numbers 1 - 105 of SEQ ID NO: 27.

[14] (e-4) The anti-PAD4 antibody or the antibody fragment thereof according to [13], wherein a heavy chain variable region consists of the amino acid sequence of amino acid numbers 1 - 120 of SEQ ID NO: 28, and a light chain variable region consists of the amino acid sequence of amino acid numbers 1 - 105 of SEQ ID NO: 27.

[15] (e-5) The anti-PAD4 antibody or the antibody fragment thereof according to [13] or [14], wherein a heavy chain consists of SEQ ID NO: 28 and a light chain consists of SEQ ID NO: 27.

[16] The anti-PAD4 antibody or the antibody fragment thereof according to any one of [11] - [15], wherein cleavage occurring during generation is less than 3% of a total generation amount.

[17] The anti-PAD4 antibody or the antibody fragment thereof according to any one of [1] - [16], wherein the antibody is a neutralizing antibody.

[18] The anti-PAD4 antibody or the antibody fragment thereof according to any one of [1] - [17], wherein the antibody is a PAD4 binding antibody represented by SEQ ID NO: 29.

[19] A nucleic acid molecule comprising a base sequence encoding the anti-PAD4 antibody or the antibody fragment thereof according to any one of [1] - [15].

[20] A recombinant vector comprising the nucleic acid molecule according [19].

[21] A transformant comprising the recombinant vector according to [20].

[22] A pharmaceutical composition comprising the anti-PAD4 antibody or the antibody fragment thereof according to any one of [1] - [18].

[23] The pharmaceutical composition according to [22], wherein said pharmaceutical composition is a preventive agent or a therapeutic agent for rheumatoid arthritis or arthritis, systemic lupus erythematosus, lupus nephritis, or graft-versus-host disease.

[24] The pharmaceutical composition according to [22] or [23], wherein said pharmaceutical composition is an inhibiting agent of citrullination in a protein.

[25] The pharmaceutical composition according to any one of [22] - [24], wherein said pharmaceutical composition is an inhibiting agent of netosis in cells.

[26] Use of the anti-PAD4 antibody or the antibody fragment thereof according to any one of [1] - [18] or the pharmaceutical composition according to any one of [22] - [25] for preventing or treating rheumatoid arthritis or arthritis, systemic lupus erythematosus, lupus nephritis, or graft-versus-host disease.

[27] Use of the anti-PAD4 antibody or the antibody fragment thereof according to any one of [1] - [18] or the pharmaceutical composition according to any one of [22] - [25] for in manufacture of a preventive agent or a therapeutic agent for rheumatoid arthritis or arthritis, systemic lupus erythematosus, lupus nephritis, or graft-versus-host disease.

[28] A method for preventing or treating rheumatoid arthritis or arthritis, systemic lupus erythematosus, lupus nephritis, or graft-versus-host disease, said method comprising administering an effective amount of the anti-PAD4 antibody or the antibody fragment thereof according to any one of [1] - [18] or the pharmaceutical composition according to any one of [22] - [25].

ADVANTAGEOUS EFFECTS OF THE INVENTION

The present invention provides an anti-PAD4 antibody having excellent properties, and provides an excellent method for preventing or treating RA or arthritis, systemic lupus erythematosus, lupus nephritis, or graft-versus-host disease.

BRIEF DESCRIPTION OF THE DRAWINGS

Fig. 1 shows a method for preparing CAIA model mice and an effect of an anti-PAD4 antibody of the present invention on arthritis in the CAIA model.

Fig. 2 shows the effect of an anti-PAD4 antibody of the present invention on arthritis in the CIA model.

Fig. 3 shows the effect of an anti-PAD4 antibody of the present invention on urinary proteins in the cGvHD model.

Fig. 4 shows the effect of an anti-PAD4 antibody of the present invention on the incidence rate in the cGvHD model.

EMBODIMENTS FOR CARRYING OUT THE INVENTION

In order to facilitate understanding of the present invention, terms used in the present invention are described below.

[Neutralization]

In the present invention, "neutralization" means an action that can bind to a target of interest and inhibit any function of the target. That is, "neutralizing the activity of PAD4" means that an anti-PAD4 antibody inhibits the activity of PAD4 by binding to PAD4. The activity of PAD4 can be evaluated by one or more of several in vitro or in vivo analyses known in the art. The activity of PAD4 is, for example, the citrullination activity of arginine in proteins, and the neutralization activity of an anti-PAD4 antibody can be evaluated by a citrullination inhibition test described herein.

[Isolated]

"Isolated" such as in an isolated anti-PAD4 antibody means that it has been identified and isolated, and/or collected from a component in a natural state. Impurities in a natural state are substances that can interfere with diagnostic or therapeutic use of antibodies thereof, and examples thereof include enzymes, hormones and other proteinaceous or non-proteinaceous solutes. In general, to isolate an anti-PAD4 antibody, purification may be performed by at least one purification process, and an anti-PAD4 antibody purified by at least one purification process can be referred to as an "isolated anti-PAD4 antibody."

[Human antibody]

A human antibody refers to an antibody derived from human immunoglobulin along with light and heavy chains. Human antibodies include, depending on a difference in a constant region of a heavy chain, IgG (including IgG1, IgG2, IgG3 and IgG4) having a heavy chain of a $\gamma$ chain, IgM having a heavy chain of a $\mu$ chain, IgA (including IgA1 and IgA2) having a heavy chain of an $\alpha$ chain, IgD having a heavy chain of a $\delta$ chain, or IgE having a heavy chain of $\epsilon$ chain. In principle, a light chain includes either a $\kappa$ chain or a $\lambda$ chain.

[Humanized antibody]

A humanized antibody refers to an antibody consisting of a variable region and a constant region, the variable region consisting of a complementarity determining region of a non-human animal-derived antibody and a framework region derived from a human antibody, and the constant region being derived from a human antibody.

[Chimeric antibody]

A chimeric antibody refers to an antibody in which a light chain, a heavy chain, or both of them consist of a non-human-derived variable region and a human-derived constant region.

[Anti-PAD4 antibody]

In the present invention, an anti-PAD4 antibody refers to an immunoglobulin molecule or a modified molecule thereof that binds to PAD4. Modified molecules include multi-specific antibodies, chimeric antibodies, humanized antibodies, functionally modified antibodies, and conjugate antibodies.

[Multi-specific antibody]

A multi-specific antibody is an asymmetric antibody with two or more independent antigen recognition sites having two or more different antigen specificities, and examples thereof include bispecific antibodies having two antigen specificities and trispecific antibodies having three antigen specificities.

[Functionally modified antibody]

In the present invention, a functionally modified antibody refers to an antibody of which functions other than an antigen-binding function of an antibody, such as a cell killing function, a complement activation function, and a blood half-life prolonging function are modified by modifying an antibody sequence, a sugar chain, or the like.

[Conjugate antibody]

In the present invention, a conjugate antibody refers to an antibody in which a functional molecule other than an antibody such as a non-peptide polymer (such as a polyethylene glycol (PEG)), a radioactive substance, a toxin, a low molecular weight compound, a cytokine, a growth factor, an albumin, or an enzyme is bound to an antibody chemically or by genetic engineering.

[Antibody fragment]

In the present invention, an antibody fragment means an antigen-binding fragment unless otherwise specified. An antibody fragment can also be called an antigen-binding molecule. An antigen-binding fragment is a protein containing a part of an antibody and can bind to an antigen. Examples of antigen-binding fragments include $F(ab')_2$, Fab', Fab, Fv (variable fragment of antibody), disulfide-bonded Fv, single-chain antibody (scFv), and polymers thereof. Further, antigen-binding fragments include conjugate antigen-binding fragments that bind, chemically or by genetic engineering, a functional molecule other than the anti-PAD4 antibody of the present invention such as a non-peptidic polymer (such as a polyethylene glycol (PEG)), a radioactive substance, a toxin, a low molecular weight compound, a cytokine, a growth factor (such as TGF-β, NGF, or Neurotrophin), an albumin, an enzyme, or other antibodies.

[Complementarity determining region]

A complementarity determining region (CDR) refers to a region of a variable region of an immunoglobulin molecule that forms an antigen binding site, is also called a hypervariable region, and refers to a portion where a change in amino acid sequence is particularly large for each immunoglobulin molecule. There are three CDRs (LCDR1, LCDR2, LCDR3, and HCDR1, HCDR2, HCDR3) for each of a light chain and a heavy chain in a CDR. In the present invention, CDRs of immunoglobulin molecules are determined according to the Kabat numbering system (Kabat et al., 1987, Sequences of Proteins of Immunological Interest, US Department of Health and Human Services, NIH, USA).

[Percentage (%) identity of amino acid sequence]

A "percent (%) identity" regarding an identified reference polypeptide sequence such as a variable region is defined as a percentage of amino acid residues in a candidate sequence that are identical to amino acid residues of a specific reference polypeptide sequence after aligning the sequences, introducing gaps when necessary in order to obtain a maximum % identity, and not considering any conservative substitution as a part of a sequence identity. Alignment for a purpose of measuring the % identity can be achieved using various methods within skills of a person skilled in the art, for example, publicly available computer software such as BLAST, BLAST-2, ALIGN, or Megalign (DNASTAR) software. A person skilled in the art can determine appropriate parameters for aligning the sequences, including any algorithm required to achieve a maximum alignment with respect to full lengths of the sequences being compared. However, for a purpose here, a % identity value is obtained using a sequence comparison computer program BLAST in a pairwise alignment.

In a situation where BLAST is used for an amino acid sequence comparison, a % identity of a given amino acid sequence A with a given amino acid sequence B is calculated as follows:

$$100 \text{ times a ratio } X/Y$$

where X is the number of amino acid residues with scores consistent with being identical as determined by a program alignment of A and B of the sequence alignment program BLAST, and Y is the total number of amino acid residues of B. When the length of the amino acid sequence A is different from the length of the amino acid sequence B, it is understood that the % identity of A with respect to B is different from the % identity of B with respect to A. Unless otherwise noted, here, all % identity values are obtained using the BLAST computer program as described in the immediately preceding paragraph.

[Competing]

In the present invention, "competing" with the anti-PAD4 antibody of the present invention means that, when measured using a surface plasmon resonance (SPR) method described herein, due to the presence of the anti-PAD4 antibody or the antigen-binding fragment thereof, binding between the anti-PAD4 antibody of the present invention and PAD4 is significantly reduced.

In the following, the present invention is described in detail.

<PAD4>

PAD4 is generally known as an enzyme involved in citrullination of arginine in proteins. Further, PAD4 is also known as an enzyme involved in netosis in cells. Details of an amino acid sequence or the like of PAD4 can be found on websites of NCBI (National Center for Biotechnology Information), HGNC (HUGO Gene Nomenclature Committee), and the like. An accession number for PAD4 described in NCBI is, for example, NP_036519.2. The amino acid sequence of PAD4 is, for example, SEQ ID NO: 29. As long as PAD4 has the PAD4 activity, a biological origin of PAD4 is not limited.

<anti-PAD4 antibody>

An embodiment of the present invention is a novel anti-PAD4 antibody. This antibody is an anti-PAD4 antibody that has better binding property, storage stability, and chemical stability than a conventional anti-PAD4 antibody. The storage stability and the chemical stability will each be described later, but these may be collectively referred to as stability. When this antibody is used, for example, RA or arthritis, systemic lupus erythematosus, lupus nephritis, or graft-versus-host disease can be prevented or treated. This preventive or therapeutic method uses an antibody, and thus has few side effects and is excellent from a point of view of safety.

Further, the anti-PAD4 antibody according to the embodiment of the present invention may be an antibody that suppresses the citrullination activity of a protein due to PAD4.
Further, the anti-PAD4 antibody according to the embodiment of the present invention may be an antibody that suppresses netosis in cells.

The anti-PAD4 antibody according to the embodiment of the present invention has an excellent binding property to PAD4. The binding of the anti-PAD4 antibody of the present invention to PAD4 means a PAD4-specific binding. However, those with a low dissociation constant (KD) with respect to PAD4 (preferably human PAD4) are even more preferred. An upper limit is, for example, 100 pM or less, more preferably 90 pM or less, and even more preferably 80 pM or less. A lower limit is not particularly limited, but can be, for example, 20 pM or more, more preferably 30 pM or more, and more preferably 40 pM or more. The dissociation constant (KD) is, for example, a value measured using the surface plasmon resonance (SPR) method described herein.

The anti-PAD4 antibody according to the embodiment of the present invention neutralizes the anti-PAD4 antibody activity of PAD4. The neutralizing activity of the anti-PAD4 antibody can be evaluated, for example, by a citrullination inhibition test shown in an example described below. By adding the anti-PAD4 antibody, citrullination due to PAD4 is inhibited. The anti-PAD4 antibody of the present invention can neutralize the citrullination activity of PAD4 by 50% or more, more preferably 80% or more, and most preferably 90% or more.

The anti-PAD4 antibody according to the embodiment of the present invention has excellent preservation (storage) stability. The storage stability can be evaluated by a decrease in binding to PAD4 after storage for a certain period of time. Specifically, a PAD4-binding amount of the anti-PAD4 antibody can be measured by an antigen binding activity measurement using a surface plasmon resonance apparatus described in Example 5. The anti-PAD4 antibody according to the embodiment of the present invention has a PAD4-binding amount of 90%, 93%, 94%, 95%, 96%, 97% or more when the anti-PAD4 antibody or the antibody fragment thereof is stored at 40 °C for one month as compared to a PAD4-binding amount of the anti-PAD4 antibody or the antibody fragment thereof before the storage.

A binding site when the anti-PAD4 antibody of the present invention binds to PAD4 is not particularly limited, but for example, it preferably binds to an epitope containing positions 345, 347, and 348 of PAD4 (for example, SEQ ID NO: 29).

The anti-PAD4 antibody of the present invention includes: a monoclonal antibody obtained by producing a hybridoma by using PAD4 or a partial fragment thereof as an antigen and immunizing a mammal (such as a mouse) or a chicken with the antigen; chimeric and humanized antibodies produced using a genetic recombination technology; a human antibody produced using a human antibody-producing transgenic animal or the like; and the like. Further, an antibody obtained by subjecting an antibody obtained above to affinity maturation is also included. For example, G8 to be described later can be used as a parent antibody. When the anti-PAD4 antibody of the present invention or an antibody fragment thereof is administered to a human as a medical drug, a humanized antibody or a human antibody or an antibody fragment thereof is desirable from a point of view of side effects.

An antigen may be used as it is for immunization, or may be used as a complex with a carrier protein. A condensing agent such as glutaraldehyde, carbodiimide, or maleimide active ester can be used in preparing a complex of an antigen and a carrier protein. Examples of carrier proteins include bovine serum albumin, thyroglobulin, hemocyanin, KLH, and the like.

Examples of animals to be immunized include mammals (such as mice, rats, hamsters, guinea pigs, rabbits, cats, dogs, pigs, goats, horses and cows) and chickens, and examples of inoculation methods include subcutaneous administration, muscular administration, and intraperitoneal administration. Administration may be mixed with a complete Freund's adjuvant or an incomplete Freund's adjuvant, and the administration is usually performed once every 2-5 weeks. Antibody-producing cells obtained from the spleen or lymph nodes of immunized animals are fused with myeloma cells and isolated as hybridomas. As myeloma cells, those derived from animals, for example, those derived from mice, rats, humans, chickens, and the like are used.

<Monoclonal antibody>

Specifically, a monoclonal antibody can be obtained as follows. That is, the above-described antigen is used as an immunogen, and immune sensitization is performed by injecting 1 to several times or transplanting the immunogen, with Freund's adjuvant as needed, subcutaneously, intramuscularly, intravenously, in a hood pad or intraperitoneally into an animal described above. Usually, immunization is performed 1 to 4 times about every 1 to 14 days after the initial immunization, and antibody-producing cells are obtained from the immune-sensitized animal about 1 to 5 days after the final immunization.

A monoclonal antibody can be obtained using a method commonly known to those of skill in the art (for example, "Current Protocols in Molecular Biology" (John Wiley & Sons (1987)), Antibodies: A Laboratory Manual, Ed. Harlow and David Lane, Cold Spring Harbor Laboratory (1988)).

Preparation of a "hybridoma" that secretes a monoclonal antibody can be performed according to a method of Koehler and Milstein et al. (Nature, 256, 495, 1975) and modification methods based thereon. That is, it is prepared by fusing antibody-producing cells contained in the spleen or the like obtained from an immune-sensitized animal with myeloma cells derived from an animal, preferably a mouse, a rat, a chicken or a human, which are incapable of producing autoantibodies.

As myeloma cells used for cell fusion, for example, mouse-derived myeloma P3/X63-AG8.653 (653), P3/NSI/1-Ag4-1 (NS-1), P3/X63-Ag8.U1 (P3U1), SP2/0-Ag14 (Sp2/O, Sp2), PAI, F0 or BW5147, rat-derived myeloma 210RCY3-Ag.2.3., human-derived Mieroma U-266AR1, GM1500-6TG-A1-2, UC729-6, CEM-AGR, D1R11 or CEM-T15, and the like can be used.

Examples of fusion accelerating agents include polyethylene glycol and the like. Usually, cell fusion can be performed by using polyethylene glycol at a concentration of about 20 - 50% (having an average molecular weight of 1000 - 4000) to cause a reaction for about 1 - 10 minutes under a temperature of 20 - 40 °C, preferably 30 - 37 °C with a ratio of the number of antibody-producing cells to the number of myeloma cells being usually about 1:1 - 10:1.

Screening for hybridoma clones that produce monoclonal antibodies can be performed by culturing hybridomas in, for example, microtiter plates, and measuring reactivities of well culture supernatants with immunoantigens using an immunochemical method such as ELISA.

Clones can be obtained by further cloning from wells containing hybridomas that produce antibodies of interest using a limiting dilution method. Hybridoma selection and breeding is usually performed in a medium for animal cells containing 10 - 20% of fetal bovine serum by adding HAT (hypoxanthine, aminopterin, thymidine).

Production of monoclonal antibodies from hybridomas can be performed by culturing hybridomas in vitro, or growing them in vivo in ascites of animals such as mice, rats and chickens, and isolating them from an obtained culture supernatant or the ascites of the animals.

In the case of culturing in vitro, it is possible to use a nutrient medium suitable for growing, maintaining and preserving hybridomas and producing monoclonal antibodies in a culture supernatant according to various conditions such as characteristics of a cell type to be cultured and a culture method.

Examples of a basic medium include low calcium media such as Ham'F12 medium, MCDB153 medium and low calcium MEM medium, high calcium media such as MCDB104 medium, MEM medium, D-MEM medium, RPMI1640 medium, ASF104 medium and RD medium, and the like. The basal medium can include, for example, serum, hormones, cytokines and/or various inorganic or organic substances, depending on a purpose.

The monoclonal antibodies can be isolated and purified by subjecting the above-described culture supernatant or ascites to saturated ammonium sulfate, an euglobulin precipitation method, a caproic acid method, a caprylic acid method, ion exchange chromatography (such as DEAE or DE52), affinity column chromatography such as anti-immunoglobulin column or protein A column, and the like. Specifically, the purification of the monoclonal antibodies may be performed using a known method for purifying immunoglobulin, and can be easily achieved, for example, by means such as an ammonium sulfate fractionation method, a PEG fractionation method, an ethanol fractionation method, use of anion exchangers, and affinity chromatography using PAD4.

Monoclonal antibodies can also be obtained using a phage display method. In the phage display method, phages selected from any phage antibody library are screened using an immunogen of interest, and phages having a desired binding property to the immunogen are selected. Next, an antibody-corresponding sequence contained in the phages is isolated or sequenced, and an expression vector containing a nucleic acid molecule encoding an antibody or antigen-binding domain is constructed based on the isolated sequence or determined sequence information. Then, monoclonal antibodies can be produced by culturing cell lines transfected with such an expression vector. By using a human antibody library as a phage antibody library, a human antibody having a desired binding property can be produced.

An example of a preferred embodiment of the anti-PAD4 antibody of the present invention is a chimeric antibody. An example of the "chimeric antibody" is a chimeric antibody in which a variable region is derived from immunoglobulin of non-human animals (such as mice, rats, hamsters, and chickens) and a constant region is derived from human immunoglobulin. For example, it can be produced by immunizing a mouse with an antigen, cutting out a variable region that binds to the antigen from a monoclonal antibody gene of the mouse, and binding it to an antibody constant region derived from human bone marrow. Constant regions derived from human immunoglobulin each have a unique amino acid sequence depending on isotypes such as IgG (IgG1, IgG2, IgG3, IgG4), IgM, IgA (IgA1, IgA2), IgD and IgE. However, a constant region of a recombinant chimeric antibody in the present invention may be a constant region of human immunoglobulin belonging to any isotype. Preferably, it is a constant region of human IgG. An expression vector can be prepared using a gene of a chimeric antibody thus prepared. A chimeric antibody-producing transformed cell is obtained by transforming a host cell with the expression vector, and a chimeric antibody of interest is obtained from a culture supernatant by culturing the transformed cell.

An example of another preferred embodiment of the anti-PAD4 antibody of the present invention is a humanized antibody. The "humanized antibody" in the present invention is an antibody in which only a DNA sequence of an antigen-binding site (CDR; complementarity determining region) of an antibody of a non-human animal such as a mouse is transplanted into a human antibody gene (CDR grafting). For example, it can be prepared by referring to methods described in JP H4-506458 and JP 2912618. Specifically, it means a humanized antibody in which a part of or entire CDR thereof is derived from a monoclonal antibody of a non-human animal (such as a mouse, rat, hamster, or chicken), a framework region of a variable region thereof is derived from human immunoglobulin, and a constant region thereof is derived from human immunoglobulin.

The humanized antibody in the present invention can be produced, for example, as follows. However, it is not limited to such a production method.

As a method for humanizing an antibody, various methods known in the art can be used (for example, Almagro et al., FRont Biosci., 2008 Jan 1; 13: 1619-1633), and specific examples thereof include CDR grafting (Ozaki et al., Blood., 1999 Jun 1; 93 (11): 3922-3930), Resurfacing (roguska et al., Proc Natl Acad Sci USA, 1994 Feb 1; 91 (3): 969-973), FR shuffle (Damschroder et al., Mol Immunol., 2007 Apr; 44 (11): 3049-3060, Epub 2007 Jan 22), and the like. In order to modify or improve antigen binding, an amino acid residue in a human FR region may be replaced with a corresponding residue from a CDR donor antibody. This FR substitution can be performed using a method commonly known in the art (Riechmann et al., Nature 1988 Mar 24; 332 (6162): 323-327). For example, an FR residue important for antigen binding may be identified by modeling an interaction between CDR and a FR residue. Or, an abnormal FR residue at a specific position may be identified by a sequence comparison. It may be humanized using a method described in Nishibori et al., Mol Immunol 2006 Feb; 43 (6): 634-42.

A human heavy chain gene transplanted with DNA of an isolated mouse heavy chain CDR portion is introduced into an appropriate expression vector such that it can be expressed, and, similarly, a human light chain gene transplanted with DNA of a mouse light chain CDR portion is introduced into another appropriate expression vector such that it can be expressed. Or, human heavy and light chain genes transplanted with mouse CDR can be introduced such that they can be expressed in the same expression vector. A humanized antibody-producing transformed cell is obtained by transforming a host cell with the expression vector thus prepared, and a humanized antibody of interest is obtained from a culture supernatant by culturing the transformed cell.

An example of another preferred embodiment of the anti-PAD4 antibody of the present invention is a human antibody. A human antibody is an antibody that is an immunoglobulin in which all regions including a heavy chain variable region and a heavy chain constant region as well as a light chain variable region and a light chain constant region that make up the immunoglobulin are derived from a gene encoding a human immunoglobulin, and can be produced by introducing a human antibody gene into a mouse. Specifically, for example, it can be produced in the same manner as the above-described method for producing a monoclonal antibody by immune-sensitizing a transgenic animal produced by incorporating at least a human immunoglobulin gene into a gene locus of a non-human animal such as a mouse or chicken with an antigen.

For example, a transgenic mouse producing a human antibody can be produced according to methods described in Nature Genetics, Vol. 7, p.13-21, 1994; Nature Genetics, Vol. 15, p.146-156, 1997; JP H4-504365; JP H7-509137; WO 94/25585; Nature, Vol. 368, p.856-859, 1994; and JP H6-500233. More specifically, examples thereof include HuMab (registered trademark) Mouse (Medarex, Princeton NJ), KMTM Mouse (Kirin Pharma Company, Japan), KM (FCγRIIb-KO) Mouse, and the like.

Specific examples of the monoclonal antibody of the present invention include:

(a) an anti-PAD4 antibody in which HCDR1 consists of an amino acid sequence of SEQ ID NO: 1, HCDR2 consists of an amino acid sequence of SEQ ID NO: 7, HCDR3 consists of an amino acid sequence of SEQ ID NO: 11, LCDR1 consists of an amino acid sequence of SEQ ID NO: 4, LCDR2 consists of an amino acid sequence of SEQ ID NO: 5, and LCDR3 consists of an amino acid sequence of SEQ ID NO: 13;

(b) an anti-PAD4 antibody in which HCDR1 consists of the amino acid sequence of SEQ ID NO: 1, HCDR2 consists of an amino acid sequence of SEQ ID NO: 8, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 13;

(c) an anti-PAD4 antibody in which HCDR1 consists of the amino acid sequence of SEQ ID NO: 1, HCDR2 consists of an amino acid sequence of SEQ ID NO: 9, HCDR3 consists of an amino acid sequence of SEQ ID NO: 12, LCDR1 consists of the amino acid sequence of SEQ ID NO: 4, LCDR2 consists of the amino acid sequence of SEQ ID NO: 5, and LCDR3 consists of an amino acid sequence of SEQ ID NO: 14;

(d) an anti-PAD4 antibody in which HCDR1 consists of the amino acid sequence of SEQ ID NO: 1, HCDR2 consists of an amino acid sequence of SEQ ID NO: 10, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of an amino acid sequence of SEQ ID NO: 15;

(e) an anti-PAD4 antibody in which HCDR1 consists of the amino acid sequence of SEQ ID NO: 1, HCDR2 consists of an amino acid sequence of SEQ ID NO: 24, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, LCDR3 consists of an amino acid sequence of SEQ ID NO: 25, and an amino acid corresponding to the 84th amino acid of a heavy chain is serine;

and the like.

The antibodies described in (a) - (d) above each have a significantly improved binding affinity with respect to PAD4. Further, the antibodies described in (a) - (d) above each have an improved storage stability of binding activity with respect to PAD4. The antibody described in (e) above has suppressed cleavage and a significantly improved chemical stability. The antibodies described in (a) - (d) above each have a high chemical stability equivalent to that of the antibody described in (e) above.

The amino acid sequences described in (a) - (e) above correspond to amino acid sequences of CDRs of antibodies of 2-1-47, 3-1-39, 3-2-37, 4-2-25, and G8ss described in Examples described below.

That is, the amino acid sequences of heavy chain CDR1, 2, 3, light chain CDR1, 2, and 3 of 2-1-47 are respectively the amino acid sequences shown by SEQ ID NO: 1, SEQ ID NO: 7, SEQ ID NO: 11, SEQ ID NO: 4, SEQ ID NO: 5, and SEQ ID NO: 13.

The amino acid sequences of heavy chain CDR1, 2, 3, light chain CDR1, 2, and 3 of 3-1-39 are respectively the amino acid sequences shown by SEQ ID NO: 1, SEQ ID NO: 8, SEQ ID NO: 11, SEQ ID NO: 4, SEQ ID NO: 5, and SEQ ID NO: 13.

The amino acid sequences of heavy chain CDR1, 2, 3, light chain CDR1, 2, and 3 of 3-2-37 are respectively the amino acid sequences shown by SEQ ID NO: 1, SEQ ID NO: 9, SEQ ID NO: 12, SEQ ID NO: 4, SEQ ID NO: 5, and SEQ ID NO: 14.

The amino acid sequences of heavy chain CDR1, 2, 3, light chain CDR1, 2, and 3 of 4-2-25 are respectively the amino acid sequences shown by SEQ ID NO: 1, SEQ ID NO: 10, SEQ ID NO: 11, SEQ ID NO: 4, SEQ ID NO: 5, and SEQ ID NO: 15.

The amino acid sequences of heavy chain CDR1, 2, 3, light chain CDR1, 2, and 3 of G8ss are respectively the amino acid sequences shown by SEQ ID NO: 1, SEQ ID NO: 24, SEQ ID NO: 11, SEQ ID NO: 4, SEQ ID NO: 5, and SEQ ID NO: 25.

As long as the anti-PAD4 antibody of (a) above maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the anti-PAD4 antibody of (a) above may comprise the amino acid sequences shown by SEQ ID NO: 1, SEQ ID NO: 7, SEQ ID NO: 11, SEQ ID NO: 4, SEQ ID NO: 5, and SEQ ID NO: 13 respectively as the amino acid sequences of the heavy chain CDR1, 2, 3, light chain CDR1, 2, and 3.

As long as the anti-PAD4 antibody of (b) above maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the anti-PAD4 antibody of (b) above may comprise the amino acid sequences shown by SEQ ID NO: 1, SEQ ID NO: 8, SEQ ID NO: 11, SEQ ID NO: 4, SEQ ID NO: 5, and SEQ ID NO: 13 respectively as the amino acid sequences of the heavy chain CDR1, 2, 3, light chain CDR1, 2, and 3.

As long as the anti-PAD4 antibody of (c) above maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the anti-PAD4 antibody of (c) above may comprise the amino acid sequences shown by SEQ ID NO: 1, SEQ ID NO: 9, SEQ ID NO: 12, SEQ ID NO: 4, SEQ ID NO: 5, and SEQ ID NO: 14 respectively as the amino acid sequences of the heavy chain CDR1, 2, 3, light chain CDR1, 2, and 3.

As long as the anti-PAD4 antibody of (d) above maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the amino acid sequences of the heavy chain CDR1, 2, 3, light chain CDR1, 2, and 3 may respectively comprise the amino acid sequences shown by SEQ ID NO: 1, SEQ ID NO: 10, SEQ ID NO: 11, SEQ ID NO: 4, SEQ ID NO: 5, and SEQ ID NO: 15.

As long as the anti-PAD4 antibody of (e) above maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the anti-PAD4 antibody of (e) above may comprise the amino acid sequences shown by SEQ ID NO: 1, SEQ ID NO: 24, SEQ ID NO: 11, SEQ ID NO: 4, SEQ ID NO: 5, and SEQ ID NO: 25 respectively as the amino acid sequences of the heavy chain CDR1, 2, 3, light chain CDR1, 2, and 3.

As long as the anti-PAD4 antibody according to the embodiment of the present invention maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the heavy chain CDR1 may comprise the amino acid sequence shown by SEQ ID NO: 1, the heavy chain CDR2 may comprise the amino acid sequence shown by SEQ ID NO: 2, the heavy chain CDR3 may comprise the amino acid sequence shown by SEQ ID NO: 3, the light chain CDR1 may comprise the amino acid sequence shown by SEQ ID NO: 4, the light chain CDR2 may comprise the amino acid sequence shown by SEQ ID NO: 5, and the light chain CDR3 may comprise the amino acid sequence shown by SEQ ID NO: 6.

In the amino acid sequence shown by SEQ ID NO: 2, an amino acid at position 11 is Tyr, Thr or Ile, an amino acid at position 12 is Gly, Ser or Pro, an amino acid at position 13 is Thr, Val, Tyr or Pro, an amino acid at position 14 is Pro or Asn, an amino acid at position 15 is Tyr, Ala, Gln or Leu, an amino acid at position 17 is Gly, Thr or Ser, the amino acids at the positions each comprise an amino acid sequence that is any combination selected from the above amino acids.

In the amino acid sequence shown by SEQ ID NO: 3, an amino acid at position 1 is Ala or Gly, and specifically comprises the amino acid sequences of SEQ ID NOs: 11 and 12. In the amino acid sequence shown by SEQ ID NO: 6, an amino acid at position 4 is Thr, Leu or Tyr, and specifically comprises the amino acid sequences of SEQ ID NOs: 13, 14 and 15.

As long as the anti-PAD4 antibody according to the embodiment of the present invention maintains the properties of having a binding ability to PAD4 (a KD value with respect to PAD4 is preferably 100 pM or less, more preferably 90 pM or less, and even more preferably 80 pM or less) and neutralizing the citrullination activity of PAD4, the heavy chain CDR1 may comprise the amino acid sequence shown by SEQ ID NO: 1, the heavy chain CDR2 may comprise the amino acid sequence shown by any one of SEQ ID NOs: 7 - 10, the heavy chain CDR3 may comprise the amino acid sequence shown by SEQ ID NO: 11 or 12, the light chain CDR1 may comprise the amino acid sequence shown by SEQ ID NO: 4, the light chain CDR2 may comprise the amino acid sequence shown by SEQ ID NO: 5, and the light chain CDR3 may comprise the amino acid sequence shown by any one of SEQ ID NOs: 13 - 15.

As long as the properties of the anti-PAD4 antibody of the present invention of having a binding ability to PAD4 (a KD value with respect to PAD4 is preferably 100 pM or less, more preferably 90 pM or less, and even more preferably 80 pM or less) and neutralizing the citrullination activity of PAD4 are maintained, in one or more of these CDRs, 1 or several amino acids may be substituted. Here, "1 or several" is, for example, 1, 2, or 3. The amino acid substitution is preferably a conservative substitution in order to maintain the properties of the present invention. Here, the "conservative substitution" means that an amino acid residue is substituted with another chemically similar amino acid residue such that an activity of a peptide is substantially not altered. Examples thereof include a case where a hydrophobic residue is substituted with another hydrophobic residue, a case where a polar residue is substituted with another polar residue having the charge, and the like. As examples of functionally similar amino acids capable of performing such substitutions, examples of non-polar (hydrophobic) amino acids include an alanine, a valine, an isoleucine, a leucine, a proline, a tryptophan, a phenylalanine, a methionine, and the like. Examples of polar (neutral) amino acids include a glycine, a serine, a threonine, a tyrosine, a glutamine, an asparagine, a cysteine, and the like. Examples of positively charged (basic) amino acids include an arginine, a histidine, a lysine, and the like. Further, examples of negatively charged (acidic) amino acids include an aspartic acid, a glutamic acid, and the like. "The properties of the antibody are maintained" means that these properties are maintained to about the same extent, for example, 80% or higher, preferably 90% or higher, and more preferably 95% or higher, as compared to those before a modification of an amino acid sequence of a CDR. Maintenance also includes improvement.

A region other than a CDR is not particularly limited as long as it is a sequence that can maintain a structure and exert a function as an antibody, and may be any one of a mouse-derived sequence, a human-derived sequence, sequences derived from other mammals, a chicken-derived sequence, chimeric sequences of these, and an artificial sequence. In a case where a constant region is included, for amino acid sequences of heavy chain and light chain constant regions, those described in Nucleic Acids Research vol.14, p1779, 1986, The Journal of Biological Chemistry vol.257, p1516, 1982, and Cell vol. 22, p197, 1980 are exemplified.

As a case where a region other than a CDR is modified, an example of a preferred embodiment of the present invention is an antibody in which an amino acid (usually asparagine) corresponding to the 84th amino acid of a heavy chain (for example, SEQ ID NO: 26) of the antibody is serine. Here, a "corresponding amino acid" refers to an amino acid (usually asparagine) present at a position of asparagine, which is the 84th amino acid of SEQ ID NO: 26, when an amino acid sequence of interest is aligned with SEQ ID NO: 26. Such an anti-PAD4 antibody of the embodiment of the present invention is excellent in antibody chemical stability, specifically, in suppressing cleavage generated during production. Cleavage suppression can be evaluated by a decrease in an amount of antibodies cleaved between the 84th asparagine residue and the 85th serine residue of a heavy chain due to peptide mapping, specifically, for example, can be evaluated by measuring an amount of cleavage peptides using a method described in Example 1. When the anti-PAD4 antibody according to the embodiment of the present invention that is excellent in suppressing cleavage generated during production is stored, for example, at 4 °C for 2 weeks, cleaved antibodies can be 3%, 2%, 1% or less compared to that before the storage.

Further, a humanized antibody in which a region other than a CDR is derived from a human is also exemplified. Examples of such humanized antibodies include one or more antibodies selected from a group consisting of:

(a) an anti-PAD4 antibody in which a heavy chain variable region and a light chain variable region respectively consist of or comprise an amino acid sequence of amino acid numbers 1 - 120 of SEQ ID NO: 16 and an amino acid sequence of amino acid numbers 1 - 105 of SEQ ID NO: 17;

(b) an anti-PAD4 antibody in which a heavy chain variable region and a light chain variable region respectively consist of or comprise an amino acid sequence of amino acid numbers 1 - 120 of SEQ ID NO: 18 and an amino acid sequence of amino acid numbers 1 - 105 of SEQ ID NO: 19;

(c) an anti-PAD4 antibody in which a heavy chain variable region and a light chain variable region respectively consist of or comprise an amino acid sequence of amino acid numbers 1 - 120 of SEQ ID NO: 20 and an amino acid sequence of amino acid numbers 1 - 105 of SEQ ID NO: 21;

(d) an anti-PAD4 antibody in which a heavy chain variable region and a light chain variable region respectively consist of or comprise an amino acid sequence of amino acid numbers 1 - 120 of SEQ ID NO: 22 and an amino acid sequence of amino acid numbers 1 - 105 of SEQ ID NO: 23; and

(e) an anti-PAD4 antibody in which a heavy chain variable region and a light chain variable region respectively consist of or comprise an amino acid sequence of amino acid numbers 1 - 120 of SEQ ID NO: 26 and an amino acid sequence of amino acid numbers 1 - 105 of SEQ ID NO: 27.

Or, the anti-PAD4 antibody according to the embodiment of the present invention may be one or more antibodies selected from a group consisting of:

(a) an anti-PAD4 antibody in which a heavy chain and a light chain respectively consist of or comprise amino acid sequences shown by SEQ ID NOs: 16 and 17;

(b) an anti-PAD4 antibody in which a heavy chain and a light chain respectively consist of or comprise amino acid sequences shown by SEQ ID NOs: 18 and 19;

(c) an anti-PAD4 antibody in which a heavy chain and a light chain respectively consist of or comprise amino acid sequences shown by SEQ ID NOs: 20 and 21;

(d) an anti-PAD4 antibody in which a heavy chain and a light chain respectively consist of or comprise amino acid sequences shown by SEQ ID NOs: 22 and 23; and

(e) an anti-PAD4 antibody in which a heavy chain and a light chain respectively consist of or comprise amino acid sequences shown by SEQ ID NOs: 26 and 27.

In a heavy chain variable region and/or a light chain variable region of an amino acid sequence of a humanized antibody, as long as properties of having a binding ability to PAD4 (a KD value with respect to PAD4 is preferably 100 pM or less, more preferably 90 pM or less, and even more preferably 80 pM or less) and neutralizing the citrullination activity of PAD4 are maintained, there may be substitution, deletion, addition or insertion of 1 or several (1 - 20, 1 - 10, or 1 - 5) amino acids. Such substitution, deletion, or addition may be introduced into a CDR, but is preferably introduced into a region other than a CDR. Further, the amino acid substitution is preferably a conservative substitution in order to maintain the properties of the present invention.

In an amino acid sequence of the anti-PAD4 antibody of the present invention including substitution, deletion, or the like in the heavy chain variable region and/or the light chain variable region, the heavy chain variable region can be an amino acid sequence having 90% or more (more preferably 95%, 96%, 97%, 98%, 99% or more) identity with the amino acid sequence of amino acid numbers 1 - 120 of SEQ ID NO: 16, the amino acid sequence of amino acid numbers 1 - 120 of SEQ ID NO: 18, the amino acid sequence of amino acid numbers 1 - 120 of SEQ ID NO: 20, the amino acid sequence of amino acid numbers 1 - 120 of SEQ ID NO: 22 or the amino acid sequence of amino acid number 1 - 120 of SEQ ID NO: 26, and the light chain variable region can be an amino acid sequence having 90% or more (more preferably 95%, 96%, 97%, 98%, 99% or more) identity with the amino acid sequence of amino acid numbers 1 - 105 of SEQ ID NO: 17, the amino acid sequence of amino acid numbers 1 - 105 of SEQ ID NO: 19, the amino acid sequence of amino acid numbers 1 - 105 of SEQ ID NO: 21, the amino acid sequence of amino acid numbers 1 - 105 of SEQ ID NO: 23 or the amino acid sequence of amino acid numbers 1 - 105 of SEQ ID NO: 27.

The anti-PAD4 antibody of the present invention further can be an anti-PAD4 antibody in which, as long as the properties of having a binding ability to PAD4 (a KD value with respect to PAD4 is preferably 100 pM or less, more preferably 90 pM or less, and even more preferably 80 pM or less) and neutralizing the citrullination activity of PAD4 are maintained, a heavy chain can be an amino acid sequence having 90% or more (more preferably 95%, 96%, 97%, 98%, 99% or more) identity with SEQ ID NOs: 16, 18, 20, 22, 26 in the sequence listing, and a light chain has an amino acid sequence having 90% or more (more preferably 95%, 96%, 97%, 98%, 99% or more) identity with SEQ ID NOs: 17, 19, 21, 23, 27 in the sequence listing.

The anti-PAD4 antibody of the present invention includes a multi-specific antibody, a functionally modified antibody or a conjugate antibody having a CDR consisting of the above specific amino acid sequence, or a variable region consisting of the above specific amino acid sequence.

By binding an antibody having an antigen-binding specificity other than PAD4 to the anti-PAD4 antibody of the present invention using a genetic engineering method, a multi-specific antibody such as a bispecific antibody can be produced. The genetic engineering method has already been established in this field. For example, by using a technology of DVD-Ig in which variable regions are linked in series (Wu et al., Nature Biotechnology 25 (11), 1290 (2007)) or ART-Ig in which heavy chains of two antibodies that bind to different antigens are combined by modifying Fc regions of the antibodies (Kitazawa et al., Nature Medicine 18 (10), 1570 (2012)), a desired bispecific antibody can be obtained.

An example of a modified molecule of the anti-PAD4 antibody of the present invention is a functionally modified antibody. A functionally modified antibody means an antibody in which, by modifying an antibody sequence, a sugar chain, or the like, functions such as a cell killing function, a complement activation function, and a blood half-life prolonging function are modified (Kenya Shitara, Journal of Pharmacy, 2009, Vol. 129 (1), p3; Akiko Ishii et al., Journal of Japanese Pharmacology, 2010, Vol. 136 (5), p280; and Shuhei Hashiguchi et al., Biochemistry, 2010, Vol. 82 (8), p710).

A function-modified antibody of the anti-PAD4 antibody is prepared using the following method. For example, when the anti-PAD4 antibody of the present invention is produced using CHO cells, in which a $\alpha$1,6-fucose transferase (FUT8) gene is disrupted, as host cells, an antibody with a reduced fucose content of a sugar chain and an enhanced cell-killing function is obtained, and when CHO cells into which the FUT8 gene has been introduced are produced as host cells, an antibody with a low cell killing function is obtained (WO 2005/035586, WO 2002/31140, and WO 00/61739). Further, the complement activation function can be regulated by modifying an amino acid residue of the Fc region (US 6737056, US 7297775, and US 7317091). Further, by using a mutant of the Fc region with enhanced binding to FcRn, which is one of Fc receptors, a blood half-life can be prolonged (Shuhei Hashiguchi, Biochemistry, 2010, Vol. 82 (8), p710). These functionally modified antibodies can be produced by genetic engineering.

An example of a modified molecule of the anti-PAD4 antibody of the present invention is a conjugate antibody. Examples of conjugate antibodies include conjugate antibodies in which a functional molecule other than the anti-PAD4 antibody of the present invention such as a non-peptidic polymer (such as a polyethylene glycol (PEG)), a radioactive substance, a toxin, a low molecular weight compound, a cytokine, a growth factor, an albumin, an enzyme, or another antibody is bound to an anti-PAD4 antibody chemically or by genetic engineering.

When PEG is bound as a functional molecule, it may be one that is commonly used, and may be a linear type or a branch type. PEG can bind to an amino group or the like of an antibody, for example, by using an NHS active group.

When a radioactive substance is used as a functional molecule, $^{131}$I, $^{125}$I, $^{90}$Y, $^{64}$Cu, $^{99}$Tc, $^{77}$Lu, $^{211}$At, or the like is used. A radioactive substance can be directly bound to an antibody using the Chloramine T method or the like.

When an enzyme is used as a functional molecule, luciferase (such as firefly luciferase and bacterial luciferase; US 4737456), malate dehydrogenase, urease, peroxidase (such as horseradish peroxidase (HRPO)), alkaline phosphatase, β-galactosidase, glucoamylase, lysozyme, saccharide oxidase (such as glucose oxidase, galactose oxidase, and glucose-6-phosphate dehydrogenase), heterocyclic oxidase (such as uricase and xanthine oxidase), lactoperoxidase, microperoxidase, and the like are used.

Examples of linker used when a toxin, a low molecular weight compound or an enzyme is chemically bound include divalent radicals (such as alkylene, arylene, and heteroarylene), linkers represented by -(CR$_2$)nO(CR$_2$)n- (where R is any substituent group, and n is a positive integer) or alkoxy repeating units (such as polyethyleneoxy, PEG, and polymethyleneoxy) and alkylamino (such as polyethyleneamino, and Jeffamine TM), and, diacid esters and amides (examples thereof include succinate, succinamide, diglycolate, malonate, caproamide, and the like). Chemical modification methods for binding functional molecules have already been established in this field (D. J. King, Applications and Engineering of Monoclonal antibodies., 1998 T. J. International Ltd, Monoclonal Antibody-Based Therapy of Cancer., 1998 Marcel Dekker Inc; Chari et al., Cancer Res., 1992 Vol. 152:127; Liu et al., Proc Natl Acad Sci USA., 1996 Vol. 93: 8681).

An "antigen-binding fragment" of an antibody in the present invention means a region of an antigen-binding portion of an antibody as described above, and specific examples thereof include F(ab')$_2$, Fab', Fab, Fv (variable fragment of an antibody), disulfide bond Fv, single-chain antibody (scFv), polymers of these, and the like, and further, antigen-binding fragments include conjugate antigen-binding fragments that bind, chemically or by genetic engineering, a functional molecule other than the anti-PAD4 antibody of the present invention such as a non-peptidic polymer (such as a polyethylene glycol (PEG)), a radioactive substance, a toxin, a low molecular weight compound, a cytokine, a growth factor (such as TGF-β, NGF, or Neurotrophin), an albumin, an enzyme, or other antibodies.

Here, "F(ab')$_2$" and "Fab" mean antibody fragments that are produced by treating immunoglobulin with proteolytic enzyme pepsin, papain, or the like, and are generated by digestion before and after a disulfide bond present between two heavy chains in a hinge region. For example, when IgG is treated with papain, it is cleaved on an upstream side of a disulfide bond present between two heavy chains in a hinge region, and two homologous antibody fragments can be produced in which a light chain fragment consisting of VL a (light chain variable region) and CL (a light chain constant region) and a heavy chain fragment consisting of VH (a heavy chain variable region) and CHγ1 (a γ1 region in a heavy chain constant region) are bound by a disulfide bond in a C-terminal region. These two homologous antibody fragments are each called Fab. Further, when IgG is treated with pepsin, it is cleaved on a downstream side of the disulfide bond present between the two heavy chains in the hinge region, and an antibody fragment can be obtained that is slightly larger than that in which the above two Fab's are linked in the hinge region. This antibody fragment is called F(ab')$_2$.

An example of a modified molecule of the antigen-binding fragment of the anti-PAD4 antibody of the present invention is a conjugate antigen-binding fragment. An example of the conjugate antigen-binding fragment is a conjugate antigen-binding fragment in which a functional molecule other than the anti-PAD4 antibody of the present invention such as a non-peptidic polymer (such as a polyethylene glycol (PEG)), a radioactive substance, a toxin, a low molecular weight compound, a cytokine, a growth factor, an albumin, an enzyme, or another antibody is bound to a region of an antigen-binding portion of the anti-PAD4 antibody chemically or by genetic engineering.

When PEG is bound as a functional molecule, it may be one that is commonly used, and may be a linear type or a branch type. PEG can bind to an amino group or the like of the anti-PAD4 antibody, for example, by using an NHS active group.

When a radioactive substance is used as a functional molecule, $^{131}$I, $^{125}$I, $^{90}$Y, $^{64}$Cu, $^{99}$Tc, [77]Lu, $^{211}$At, or the like is used. A radioactive substance can be directly bound to a region of an antigen-binding portion of the anti-PAD4 antibody using the Chloramine T method or the like.

When an enzyme is used as a functional molecule, luciferase (such as firefly luciferase and bacterial luciferase; US 4737456), malate dehydrogenase, urease, peroxidase (such as horseradish peroxidase (HRPO)), alkaline phosphatase, β-galactosidase, glucoamylase, lysozyme, saccharide oxidase (such as glucose oxidase, galactose oxidase, and glucose-6-phosphate dehydrogenase), heterocyclic oxidase (such as uricase and xanthine oxidase), lactoperoxidase, microperoxidase, and the like are used.

Examples of linker used when a toxin, a low molecular weight compound or an enzyme is chemically bound include divalent radicals (such as alkylene, arylene, and heteroarylene), linkers represented by -(CR$_2$)nO(CR$_2$)n- (where R is any substituent group, and n is a positive integer) or alkoxy repeating units (such as polyethyleneoxy, PEG, and polymethyleneoxy) and alkylamino (such as polyethyleneamino, and Jeffamine TM), and, diacid esters and amides (examples thereof include succinate, succinamide, diglycolate, malonate, caproamide, and the like). Chemical modification methods for binding functional molecules have already been established in this field (D.J.King., Applications and Engineering of Monoclonal antibodies., 1998 T.J.International Ltd, Monoclonal Antibody-Based Therapy of Cancer., 1998 Marcel Dekker Inc; Chari et al., Cancer Res., 1992 Vol. 152:127; Liu et al., Proc Natl Acad Sci USA., 1996 Vol. 93: 8681).

For the anti-PAD4 antibody of the present invention containing a CDR or a variable region having a specific amino acid sequence, from a point of view of maintaining a long blood half-life, the constant region is preferably a constant region of human IgG (IgG1, IgG2, IgG3, IgG4).

In the anti-PAD4 antibody of the present invention and an antigen-binding fragment thereof, as long as the properties of having a binding ability to PAD4 (a KD value with respect to PAD4 is preferably 100 pM or less, more preferably 90 pM or less, and even more preferably 80 pM or less) and neutralizing the citrullination activity of PAD4 are maintained, an anti-PAD4 antibody that competes for binding to PAD4 with an antibody having the specific CDR amino acid sequence as described above, and an antigen-binding fragment thereof, are also included. An example of an antibody that competes for binding to PAD4 with an antibody having the specific CDR amino acid sequence as described above is an antibody having an epitope in a region containing positions 345, 347, and 348 of PAD4. However, a G8 antibody is not included.

This antibody can be obtained (screened) and evaluated by being allowed to coexist in a binging system of an antibody having the CDR sequence as described above and PAD4. For example, it can be obtained by screening using a surface plasmon resonance (SPR) method described in WO 2016/175236.

An anti-PAD4 antibody that competes for binding to PAD4 with respect to an anti-PAD4 antibody comprising the above specific CDR amino acid sequence may be any animal-derived antibody such as a mouse antibody, a human antibody, a rat antibody, a rabbit antibody, a goat antibody, or a camel antibody, and may be a chimeric antibody or a humanized antibody, which is a combination of these antibodies. However, it is preferably a chimeric antibody, a humanized antibody, or a human antibody.

The anti-PAD4 antibody of the present invention or an antibody fragment thereof can be produced, for example, using a transformant (host) cell containing a recombinant vector containing the following nucleic acid molecule encoding the anti-PAD4 antibody of the present invention or an antibody fragment thereof.

<Nucleic acid molecule>

An embodiment of the present invention is a nucleic acid molecule that is a polynucleotide encoding the anti-PAD4 antibody of the present invention or antibody fragment thereof. The nucleic acid molecule is not particularly limited as long as it is a nucleic acid molecule encoding a polypeptide comprising the above-described CDR, variable region, or full-length amino acid sequence, and examples thereof include polynucleotides comprising a base sequence encoding each of:

(a) an amino acid sequence of amino acid numbers 1 - 120 of SEQ ID NO: 16 and an amino acid sequence of amino acid numbers 1 - 105 of SEQ ID NO: 17, which are respectively a heavy chain variable region and a light chain variable region;

(b) an amino acid sequence of amino acid numbers 1 - 120 of SEQ ID NO: 18 and an amino acid sequence of amino acid numbers 1 - 105 of SEQ ID NO: 19, which are respectively a heavy chain variable region and a light chain variable region;

(c) an amino acid sequence of amino acid numbers 1 - 120 of SEQ ID NO: 20 and an amino acid sequence of amino acid numbers 1 - 105 of SEQ ID NO: 21, which are respectively a heavy chain variable region and a light chain variable region;

(d) an amino acid sequence of amino acid numbers 1 - 120 of SEQ ID NO: 22 and an amino acid sequence of amino acid numbers 1 - 105 of SEQ ID NO: 23, which are respectively a heavy chain variable region and a light chain variable region; and

(e) an amino acid sequence of amino acid numbers 1 - 120 of SEQ ID NO: 28 and an amino acid sequence of amino acid numbers 1 - 105 of SEQ ID NO: 27, which are respectively a heavy chain variable region and a light chain variable region.

Another example of the nucleic acid molecule of the present invention is a polynucleotide comprising a base sequence encoding each of:

(a) amino acid sequences shown by SEQ ID NOs: 16 and 17, which are respectively a heavy chain and a light chain;

(b) amino acid sequences shown by SEQ ID NOs: 18 and 19, which are respectively a heavy chain and a light chain;

(c) amino acid sequences shown by SEQ ID NOs: 20 and 21, which are respectively a heavy chain and a light chain;

(d) amino acid sequences shown by SEQ ID NOs: 22 and 23, which are respectively a heavy chain and a light chain;

and

(e) amino acid sequences shown by SEQ ID NOs: 28 and 27, which are respectively a heavy chain and a light chain.

Other examples of the nucleic acid molecule of the present invention include:

a polynucleotide comprising a base sequence shown by SEQ ID NO: 32, which is a full length of a heavy chain of the anti-PAD4 antibody, and a base sequence shown by SEQ ID NO: 33, which is a full length of a light chain of the anti-PAD4 antibody;

a polynucleotide comprising a base sequence shown by SEQ ID NO: 34, which is a full length of a heavy chain of the anti-PAD4 antibody, and a base sequence shown by SEQ ID NO: 35, which is a full length of a light chain of the anti-PAD4 antibody;

a polynucleotide comprising a base sequence shown by SEQ ID NO: 36, which is a full length of a heavy chain of the anti-PAD4 antibody, and a base sequence shown by SEQ ID NO: 37, which is a full length of a light chain of the anti-PAD4 antibody;

a polynucleotide comprising a base sequence shown by SEQ ID NO: 38, which is a full length of a heavy chain of the anti-PAD4 antibody, and a base sequence shown by SEQ ID NO: 39, which is a full length of a light chain of the anti-PAD4 antibody; and

a polynucleotide comprising a base sequence shown by SEQ ID NO: 40, which is a full length of a heavy chain of the anti-PAD4 antibody, and a base sequence shown by SEQ ID NO: 31, which is a full length of a light chain of the anti-PAD4 antibody.

As long as the nucleic acid molecule of the present invention encodes a monoclonal antibody that has a binding ability to PAD4 and neutralizes the citrullination activity of PAD4, the nucleic acid molecule may include a polynucleotide that hybridizes under a stringent condition to a complementary strand DNA of a base sequence of a heavy chain variable region or a light chain variable region. Here, an example of the stringent condition is a condition of performing washing at a salt concentration corresponding 68 °C, $0.1 \times$ SSC, 0.1%SDS after Southern hybridization.

The nucleic acid molecule of the present invention may encode all of the constant regions and the variable regions of the heavy chain and the light chain, or may encode only the variable regions of the heavy chain and the light chain. The base sequences of the constant regions of the heavy chain and the light chain in the case of encoding all the constant regions and the variable regions are preferably those described in Nucleic Acids Research vol. 14, p1779, 1986, The Journal of Biological Chemistry vol. 257, p1516, 1982, and Cell vol. 22, p197, 1980.

Base sequences encoding full lengths of a heavy chain and a light chain of a humanized anti-PAD4 antibody G8 described in WO 2016/143753 are shown in SEQ ID NOs: 30 and 31. For example, by modifying this base sequence, it is possible to obtain a nucleic acid molecule that encodes the anti-PAD4 antibody of the present invention or an antibody fragment thereof.

Further, the nucleic acid molecule of the present invention can be obtained, for example, using the following method. First, a total RNA is prepared from cells such as hybridomas using a commercially available RNA extraction kit, and cDNA is synthesized by reverse transcriptase using random primers and the like. Next, in variable regions of known human antibody heavy chain gene and light chain gene, cDNA encoding an antibody is amplified using a PCR method in which oligonucleotides of conserved sequences are used as primers. A sequence encoding a constant region can be obtained by amplifying a known sequence using the PCR method. A base sequence of DNA can be determined using a conventional method, such as incorporating it into a sequencing plasmid.

Or, a DNA encoding the monoclonal antibody of the present invention can also be obtained by chemically synthesizing a sequence of a variable region or a part thereof and binding it to a sequence containing a constant region.

The present invention further provides a recombinant vector containing the nucleic acid molecule of the present invention and a transformant (host cell) containing the recombinant vector.

The recombinant vector may be a vector (for example, pBR322, pUC119 or derivatives thereof) that can be expressed in prokaryotic cells such as Echerichia coli, but a vector that can be expressed in eukaryotic cells is preferable, and a vector that can be expressed in mammal-derived cells is more preferable. Examples of the vector that can be expressed in mammal-derived cells include plasmid vectors such as pcDNA3.1 (manufactured by Invitrogen), pConPlus, pcDM8, pcDNA I/Amp, pcDNA3.1, and pREP4, and viral vectors such as pDON-AI DNA (manufactured by Takara Bio Inc.). It may be one vector containing a heavy chain coding sequence and a light chain coding sequence, or two vectors including a vector containing a heavy chain coding sequence and a vector containing a light chain coding sequence.

The transformant into which the recombinant vector of the present invention is introduced may be a prokaryotic cell such as Escherichia coli or Bacillus subtilis, but eukaryotic cells are preferable, and mammal-derived cells are more preferable. Examples of mammal-derived cells include Chinese hamster ovary cells (CHO cells), COS, myeloma, BHK, HeLa, Vero, 293, NS0, Namalwa, YB2/0, and the like.

Anti-PAD4 antibodies or antigen-binding fragments thereof obtained using a method described herein or a commonly known method can be purified until they are homogeneous. For separation and purification of antibodies or the like, separation and purification methods used for ordinary proteins may be used. For example, antibodies can be separated and purified by appropriately selecting or combining chromatography column such as affinity chromatography, a filter, extrafiltration, salting out, SDS polyacrylamide gel electrophoresis, isoelectric focusing, or the like (Antibodies: A Laboratory Manual., Ed Harlow and David Lane, Cold Spring Harbor Laboratory, 1988). However, the present invention is not limited to these. Examples of the column used for affinity chromatography include a protein A column, a protein G column, an anti-immunoglobulin antibody-binding column, an antigen-binding column, and the like. Example of the protein A column include Hyper D, POROS, Sepharose F. F. (Amersham Biosciences), and the like.

<Composition>

An embodiment of the present invention is a composition containing the above anti-PAD4 antibody or an antibody fragment thereof according to the embodiment of the present invention. When this composition is used, PAD4 can be efficiently detected. Further, citrulinization of proteins due to PAD4 can be efficiently suppressed.. Further, netosis in cells can be efficiently suppressed. Further, RA or arthritis, systemic lupus erythematosus, lupus nephritis, or graft-versus-host disease can be prevented or treated. Components contained in this composition are not particularly limited as long as the anti-PAD4 antibody or an antibody fragment thereof according to the embodiment of the present invention is contained, and for example, a buffer solution may be contained. One or more of various embodiments (such as that a carrier may be contained) related to an inhibiting agent and a pharmaceutical composition to be described later may be applied to this composition.

An embodiment of the present invention is an inhibiting agent for citrullination of proteins due to PAD4, the inhibiting agent containing the above anti-PAD4 antibody or an antibody fragment thereof according to the embodiment of the present invention. When this inhibiting agent is used, citrullination of proteins due to PAD4 can be efficiently suppressed. A rate of decrease in citrullination activity due to the above inhibiting agent may be 20, 30, 40, 60, 80% or more, or may be within a range between any two of these values. This rate of reduction may be expressed, for example, as a relative ratio when a rate of decrease when PBS is used is 0%. In an embodiment of the present invention, an "agent" includes, for example, a composition used for research or treatment. The above inhibiting agent includes, for example, a preventive agent or a therapeutic agent for RA or arthritis, systemic lupus erythematosus, lupus nephritis, or graft-versus-host disease. The above inhibiting agent can be used, for example, in vitro or in vivo. The inhibiting agent may contain the above composition according to the embodiment of the present invention. An embodiment of the present invention is a method for suppressing citrullination of proteins due to PAD4, the method including a step of causing the above anti-PAD4 antibody or an antibody fragment thereof according to the embodiment of the present invention to be in contact with PAD4. An embodiment of the present invention is a method for suppressing citrullination of proteins due to PAD4, the method including a step of administering the above anti-PAD4 antibody or an antibody fragment thereof according to the embodiment of the present invention to a patient. The above suppression methods include a suppression method performed for research or treatment. An embodiment of the present invention is use of the above anti-PAD4 antibody or an antibody fragment thereof according to the embodiment of the present invention for producing an inhibiting agent for citrullination of proteins due to PAD4.

An embodiment of the present invention is an inhibiting agent of netosis in cells, the inhibiting agent containing the above anti-PAD4 antibody or an antibody fragment thereof according to the embodiment of the present invention. When this inhibiting agent is used, netosis in cells due to PAD4 can be efficiently suppressed. A rate of decrease in netosis activity in cells due to the above inhibiting agent may be 20, 30, 40, 60, 80% or more, or may be within a range between any two of these values. This rate of reduction may be expressed, for example, as a relative ratio when a rate of decrease when PBS is used is 0%. In an embodiment of the present invention, an "agent" includes, for example, a composition used for research or treatment. The above inhibiting agent includes, for example, a preventive agent or a therapeutic agent for RA or arthritis, systemic lupus erythematosus, lupus nephritis, or graft-versus-host disease. The above inhibiting agent can be used, for example, in vitro or in vivo. The inhibiting agent may contain the above composition according to the embodiment of the present invention. An embodiment of the present invention is a method for suppressing netosis in cells, the method including a step of causing the above anti-PAD4 antibody or an antibody fragment thereof according to the embodiment of the present invention to be in contact with PAD4. An embodiment of the present invention is a method for suppressing netosis in cells, the method including a step of administering the above anti-PAD4 antibody or an antibody fragment thereof according to the embodiment of the present invention to a patient. The above suppression methods include a suppression method performed for research or treatment. An embodiment of the present invention is use of the above anti-PAD4 antibody or an antibody fragment thereof according to the embodiment of the present invention for producing an inhibiting agent for netosis in cells.

An embodiment of the present invention is a pharmaceutical composition containing the above anti-PAD4 antibody or an antibody fragment thereof according to the embodiment of the present invention. When this pharmaceutical composition is used, it is possible to prevent, treat, or prevent recurrence of RA or arthritis, systemic lupus erythematosus, lupus nephritis, or graft-versus-host disease. The pharmaceutical composition may contain one or more pharmacologically acceptable carriers. The pharmaceutical composition includes, for example, a pharmaceutical composition for treating RA or arthritis, systemic lupus erythematosus, lupus nephritis, or graft-versus-host disease, and a pharmaceutical composition for preventing RA or arthritis, systemic lupus erythematosus, lupus nephritis, or graft-versus-host disease. The pharmaceutical composition may contain the above composition according to the embodiment of the present invention.

An embodiment of the present invention is a method for preventing, treating or preventing recurrence of a disease, the method including a step of administering to a patient an effective amount of the above anti-PAD4 antibody or an antibody fragment thereof according to the embodiment of the present invention (or a pharmaceutical composition containing the anti-PAD4 antibody or an antibody fragment thereof). The disease is, for example, RA or arthritis, systemic lupus erythematosus, lupus nephritis, or graft-versus-host disease. An embodiment of the present invention is use of the above anti-PAD4 antibody or an antibody fragment thereof according to the embodiment of the present invention (or a pharmaceutical composition containing the anti-PAD4 antibody or an antibody fragment thereof) for preventing, treating or preventing recurrence of RA or arthritis, systemic lupus erythematosus, lupus nephritis, or graft-versus-host disease.

An embodiment of the present invention is use of the above anti-PAD4 antibody or an antibody fragment thereof according to the embodiment of the present invention (or a pharmaceutical composition containing the anti-PAD4 antibody or an antibody fragment thereof) for producing a preventive agent, a therapeutic agent or a recurrence preventive agent for RA or arthritis, systemic lupus erythematosus, lupus nephritis, or graft-versus-host disease.

In an embodiment of the present invention, an "agent" may be a pharmaceutical composition containing an active ingredient and one or more pharmacologically acceptable carriers. In an embodiment of the present invention, a "pharmaceutical composition" may be produced, for example, by mixing an active ingredient with the above carriers and using any method known in the technical field of pharmaceutics. Further, a pharmaceutical composition is not limited in a form of use as long as it is used for prevention or treatment, and may be an active ingredient alone or a mixture of an active ingredient and any ingredient.

An effect of a pharmaceutical composition in treating and/or preventing RA may be evaluated, for example, by an arthritis score, an RA score, a swelling width, diagnostic imaging, a modified Total Sharp score, a Disease Activity Score (DAS), or ACR20, ACR50, ACR70 or disease marker representing an achievement rate of the rheumatoid activity evaluation criteria prepared by the American Rheumatoid Arthritis Association (ACR). In the case of evaluating by the arthritis score, for example, when the arthritis score of a patient during administration of the pharmaceutical composition is significantly decreased compared to the arthritis score without the administration, it may be determined that there is a therapeutic and/or preventive effect. Or, when the arthritis score of a patient during administration of the pharmaceutical composition is significantly decreased as compared to the arthritis score of the patient during administration of a negative control substance, it may be determined that there is a therapeutic and/or preventive effect. The above decrease may be, for example, 10, 9, 8, 7, 6, 5, 4, 3, 2 or 1 point or less, or may be within a range between any two of these values. Or, when a graph area of the arthritis score of a patient during administration of the pharmaceutical composition is significantly decreased as compared to a graph area of the arthritis score without the administration, it may be determined that there is a therapeutic and/or preventive effect. Or, when the graph area of the arthritis score of a patient during administration of the pharmaceutical composition is significantly decreased as compared to the graph area of the arthritis score of the patient during administration of a negative control substance, it may be determined that there is a therapeutic and/or preventive effect. The above decrease may be, for example, 100, 90, 80, 70, 60, 50, 40, 30, 20, 10% or less, or may be within a range between any two of these values.

An effect of a pharmaceutical composition in treating and/or preventing systemic lupus erythematosus and lupus nephritis may be evaluated, for example, by disorders in organs, a urine protein amount, a urine protein score, a survival rate, a pathology evaluation score, a blood autoantibody production amount, an amount of blood 3rd and 4th complement components (C3 and C4), or a disease marker. Further, it may be evaluated by an SLE disease activity index (SLEDAI) that is obtained by scoring these, a British Isles Lupus Assessment Group (BILAG) index that compares a disease activity with that of 1 month ago, a Physician's global assessment (PGA) that evaluates an overall disease activity of a patient by a physician, an SLE responder index (SRI) 4 that comprehensively evaluates these, and the like. In the case of evaluating by the urine protein amount, for example, when the urinary albumin level or albumin/creatinine ratio of a patient during administration of the pharmaceutical composition is significantly decreased as compared to that without the administration, it may be determined that there is a therapeutic and/or preventive effect. Or, when the urinary albumin level or albumin/creatinine ratio of a patient during administration of the pharmaceutical composition is significantly decreased as compared to the urinary albumin level or albumin/creatinine ratio of the patient during administration of a negative control substance, it may be determined that there is a therapeutic and/or preventive effect. Or, when a graph area of the urinary albumin level or albumin/creatinine ratio of a patient during administration of the pharmaceutical composition is significantly decreased as compared to the graph area of the urinary albumin level or albumin/creatinine ratio of the patient without the administration, it may be determined that there is a therapeutic and/or preventive effect. Or, when the urinary albumin level or albumin/creatinine ratio of a patient during administration of the pharmaceutical composition is significantly decreased as compared to the urinary albumin level or albumin/creatinine ratio of the patient during administration of a negative control substance, it may be determined that there is a therapeutic and/or preventive effect. The above decrease may be, for example, 100, 90, 80, 70, 60, 50, 40, 30, 20, 10% or less, or may be within a range between any two of these values.

An effect of a pharmaceutical composition in treating and/or preventing graft-versus-host disease can be evaluated, for example, by an effect in improving characteristic organ findings, or an effect in reducing steroid, or the like. For skin lesions, it can be evaluated by an improvement in polymorphic skin atrophy or scleroderma sclerotic lesions, and for liver lesions, for example, it can be evaluated by an improvement in total bilbilin, or ALP, AST, ALT. Further, for renal lesions, for example, it may be evaluated by a urine protein amount or a urinary albumin/creatinine ratio. Determination of a therapeutic and/or preventive effect in this case can be the same as in the case of SLE described above.

Here, "patient" includes humans or mammals other than humans (for example, one or more of mice, guinea pigs, hamsters, rats, mice, rabbits, pigs, sheep, goats, cows, horses, cats, dogs, marmosets, monkeys, chimpanzees, and the like). Further, the patient may also be a patient diagnosed with RA or arthritis, systemic lupus erythematosus, lupus nephritis, or graft-versus-host disease. Further, the patient may be a patient diagnosed with a disease treatable by suppressing citrullination in cells. Further, the patient may be a patient diagnosed with a disease treatable by suppressing netosis in cells.

Here, "treatment" includes any treatment of diseases of mammals, especially humans, and includes: inhibiting a disease symptom, that is, stopping its progression or eliminating the disease or symptom; and relieving a disease symptom, that is, causing a relief of a disease or symptom, or causing a delay in progression of a symptom.

Further, "prevention" includes preventing development of the above diseases in mammals, especially humans.

Further, "preventing recurrence" includes preventing recurrence of the above diseases that repeat remission and recurrence in mammals, especially humans.

The anti-PAD4 antibody or an antigen-binding fragment thereof of the present invention, or the pharmaceutical composition containing the anti-PAD4 antibody or an antibody fragment thereof is not particularly limited in form of administration, and can be administered to mammals, including humans, by either oral administration or parenteral administration (for example, intravenous injection, intramuscular injection, subcutaneous administration, rectal administration, transdermal administration, intracerebral administration, intrathecal administration, and other local administration).

Dosage forms for oral administration and parenteral administration and preparation methods thereof are commonly known to those skilled in the art, and a pharmaceutical composition can be produced by blending the antibody of the present invention with a pharmaceutically acceptable carrier and the like.

Examples of dosage forms for parenteral administration include injection formulations (such as drip injection, intravenous injection, intramuscular injection, subcutaneous injection, intradermal injection, intracerebral administration formulation, and intraspinal administration formulation), external preparations (such as ointment, poultice, and lotion), suppository inhalants, ophthalmic agents, eye ointments, nasal drops, ear drops, liposomal agents, and the like. In particular, to directly act on the central nervous system, it can be continuously injected using a medical micropump of an osmotic pump, or it can also be mixed with a fibrin glue or the like to form a sustained-release preparation, which is then placed in an affected tissue.

For example, injectable formulations are usually prepared by dissolving the antibody in distilled water for injection. However, when necessary, a dissolution aid, a buffer, a pH regulating agent, an isotonicizing agent, a soothing agent, a preservative, stabilizer, and the like can be added. Further, it can also be a lyophilized formulation for preparation at the time of use.

Examples of the dosage form for oral administration include solid or liquid dosage forms, and, specifically, include tablets, coated tablets, pills, fine granules, granules, powders, capsules, syrups, emulsions, suspensions, injectable drugs, troches, and the like.

The pharmaceutical composition of the present invention may further contain other therapeutically effective agents, and when necessary, can contain other ingredients such as bactericides, anti-inflammatory agents, vitamins, and amino acids.

Examples of the pharmaceutically acceptable carrier include an excipient, a lubricant, a binding agent and a disintegrating agent in a solid formulation, or a solvent, a solubilizing agent, a suspending agent, an isotonizing agent, a buffering agent, a soothing agent and the like in a liquid formulation. Further, when necessary, additives such as an ordinary antiseptic agent, an anti-oxidizing agent, a coloring agent, a sweetener, an adsorbent, and a wetting agent can be appropriately used in appropriate amounts.

A dose of the antibody of the present invention is determined, for example, by a physician based on various factors such as a route of administration, a type of disease, severity of symptoms, age, gender and body weight of a patient, severity of disease, pharmacological findings such as pharmacokinetics and toxicological features, whether or not a drug delivery system is used, and whether or not the antibody is administered as a part of a combination with other drugs, but usually, for each adult (having a body weight of 60 kg), for oral administration, 1 - 5,000 $\mu$g/day, preferably 10 - 2,000 $\mu$g/day, and more preferably 50 - 2,000 $\mu$g/day, and for injection administration, 1 - 5,000 $\mu$g/day, preferably 5 - 2,000 $\mu$g/day, and more preferably 50 - 2,000 $\mu$g/day can be administered in one or several divided doses. For systemic parenteral administration, per body weight, 10 - 100,000 $\mu$g/kg, more preferably 100 - 50,000 $\mu$g/kg, and even more preferably 500 - 20,000 $\mu$g/kg can be administered daily, weekly, monthly, or 1 - 7 times per year. For local administration using an osmotic pump or the like, usually, for each adult (having a body weight of 60 kg), it can be continuously injected at a rate of 10 - 100,000 $\mu$g/day, more preferably 100 - 10,000 $\mu$g/day, and even more preferably 500 - 5,000 $\mu$g/day.

EXAMPLES

In the following, the present invention is described in more detail with reference to Examples. However, the scope of the present invention is not limited to these Examples.

<Example 1> Confirmation of chemical stability of anti-PAD4 antibody

Chemical stability of a variable region containing a CDR of a humanized anti-PAD4 antibody G8 described in WO 2016/143753 was evaluated. An expression vector for mammalian cells expressing IgG was constructed by inserting DNA encoding amino acid sequences of a light chain and a heavy chain of a test antibody G8 on a downstream side of a CMV promoter. For the DNA sequences of the light chain and the heavy chain, SEQ ID NOs: 27 and 26 in the sequence listing were respectively used. The above expression vector was introduced into ExpiCHO (Life Technologies) using a gene transfer reagent ExpiFectamine CHO Transfection Kit (Life Technologies). After culturing for 14 days after gene transfer, a culture supernatant was obtained. Using an open column, IgG was purified from the culture supernatant by affinity chromatography using a Protein A resin (GE Healthcare, MabSelect SuRe). IgG bound to the Protein A resin was washed with PBS (pH 7.2) of 6 column volume. Then, it was eluted with Arg-Antibody Elution Buffer (Nacalai) of pH 4.0 and neutralized promptly to bring the pH to near neutral. For a purpose of increasing purification purity, IgG after the protein A column purification was purified with CHT (ceramic hydroxyapatite Type II resin) (Bio-rad) using AKTA prime plus (GE Healthcare). IgG bound to CHT was eluted with gradient of a NaCl concentration, a desired fraction was collected, and then solution substitution with PBS (pH 7.4) was performed using a gel filtration chromatography method using PD-10 (GE Healthcare). The test antibody was concentrated to a concentration of about 10 mg/mL and stored at 4 °C or 37 °C for 2 weeks.

The chemical stability of the variable region containing the CDR of the test antibody G8 was evaluated by a peptide mapping analysis using liquid chromatography/mass spectrometry (LC-MS). The test antibody was subjected to a reduction treatment by reacting with a reducing agent dithiothreitol (Wako Pure Chemical Industries, Ltd.) at 37 °C for 2 hours in the presence of guanidine hydrochloride (Wako Pure Chemical Industries, Ltd.). A thiol group generated in a reduction reaction was alkylated by adding an alkylating agent iodoacetamide (Wako Pure Chemical Industries, Ltd.) to cause a reaction for 30 minutes. Next, the reaction solution was buffer-exchanged using a desalting column Zeba Spin column (Thermo Fisher Scientific), and was subjected to a reaction overnight at 37 °C under the conditions of 2 mol/L urea (SIGMA), 0.1 mol/L $NH_4HCO_3$ (Wako Pure Chemical Industries, Ltd.), 1 mmol/L $CaCl_2$ (Wako Pure Chemical Industries, Ltd.), and 0.8 $\mu$g trypsin (Wako Pure Chemical Industries, Ltd.). An obtained enzyme digested product was used as an LC-MS measurement sample.

Peptides obtained by trypsin digestion were separated by reverse phase chromatography, and a peptide mapping analysis was performed by acquiring MS and MS/MS spectra with a mass spectrometer.

LC was performed with the following parameters.

Device: ACQUITY UPLC (Waters)
Mobile phase: Buffer A=ultrapure water containing 0.1% formic acid; and Buffer B =acetonitrile containing 0.1% formic acid
Separation method: Linear gradient separation (%B = 1 to 40 in 60 minutes)
Flow rate: 0.2 mL/min
Column: ACQUITY UPLC Peptide BEH C18 column, 300 Angstrom, 1.7 $\mu$m, 2.1x150 mm (Waters)
Column oven temperature: 40 °C
Sample volume: 30 $\mu$L

Detection wavelength: 215 nm

MS and MS/MS were performed with the following parameters.

Device: Synapt (Waters)

TOF mode: V mode

Ionization method: ESI Positive

MS measurement range: 50 - 2,000 Da

MS cone voltage: 24 V

Scan time: 0.4 s

MS/MS method: MSE

Trap CE voltage: Low energy 6V, High energy ramp 20 - 40 V

Obtained mass data was analyzed using Biopharma Lynx Ver. 1.3.3 (Waters).

A post-translational modification ratio was calculated using the following method. First, peptides matching the sequence of the test antibody were extracted using Biopharma Lynx, and peptides that can be obtained by MS/MS (MS/MS b/y ion found $\geqq$ 2) were analyzed. The post-translational modification ratio was calculated from a ratio of an MS intensity of peptides containing post-translational modifications to an MS intensity of all detected peptides.

Peptides cleaved between the 84th asparagine residue and the 85th serine residue in a framework region of a heavy chain were identified by peptide mapping on a sample stored at 4 °C after purification of the test antibody G8 and a sample stored at 37 °C for 2 weeks. In the sample stored at 4 °C after purification, a ratio of cleaved peptides was as high as 4.6% of peptides containing the amino acid, indicating that the site may be potentially cleaved. Further, no cleavage was identified between other asparagine residue and serine residue in the test antibody, and it was specific between the 84th asparagine residue and the 85th serine residue.

Since it is cleaved between the asparagine residue and the serine residue, a humanized anti-PAD4 antibody G8ss was prepared by substituting the 84th asparagine residue with a serine residue. Peptide mapping was performed on a sample stored at 4 °C after purification of the test antibody G8ss and a sample stored at 40 °C for 2 weeks. As a result, no peptide cleaved between the 84th serine residue and the 85th serine residue was detected under either storage condition. From the above, it is considered that the humanized anti-PAD4 antibody has a higher chemical stability when the 84th is a serine residue.

Binding affinities of the test antibodies G8 and G8ss with respect to human PAD4 proteins were determined by measuring a dissociation constant ($KD$) in HBS-EP+ using a surface plasmon resonance apparatus Biacore T200 (GE Healthcare). A biotin-labeled human PAD4 protein was immobilized on a sensor chip using a Biotin Capture Kit (GE Healthcare). The PAD4 protein was prepared to 0.25 $\mu$g/mL using HBS-EP+ and used as a ligand solution. The ligand solution was added to a flow cell at a flow rate of 10 $\mu$L/min for 30 seconds, and then HBS-EP+ was added for 60 seconds. A flow cell to which no ligand solution was added was used as a reference cell. The test antibody was prepared from several hundred pM to several nM using HBS-EP+ and used as an analyte solution. Further, a running buffer solution was used as a blank solution. The blank solution or the analyte solution was added at a flow rate of 30 $\mu$L/min for 180 seconds using a single-cycle method, and a dissociation time was 1200 seconds. A sensorgram of the reference cell is subtracted from a sensorgram of the flow cell to which the ligand was added when the blank solution or the analyte solution was added, and further, a sensorgram to which the blank solution was added was subtracted from the sensorgram to which the analyte solution was added, and the result was used for analysis. Binding parameters were calculated using a 1:1 binding model using Biacore T200 Evaluation software (GE Healthcare). The test antibody G8 bound to a human PAD4 protein with a binding affinity having a binding rate constant $k_{on}$ = 7.70$\times$10$^6$ (1/Ms), a dissociation rate constant $k_{off}$ = 1.65$\times$10$^{-3}$ (1/s), and a dissociation constant KD = 214 pM. The test antibody G8ss bound to a human PAD4 protein with a binding affinity having a binding rate constant $k_{on}$ = 6.37$\times$10$^6$ (1/Ms), a dissociation rate constant $k_{off}$ = 1.76$\times$10$^{-3}$ (1/s), and a dissociation constant $KD$ = 276 pM. From the above, it was found that in the humanized anti-PAD4 antibody, the binding affinity to human PAD4 does not change even when the 84th is replaced with a serine residue.

<Example 2> Determination of amino acid substitution site for improving complementarity determining region

For a purpose of improving the affinity to human PAD4 and functionality of the humanized anti-human PAD4 antibody G8, improvement of a complementarity determining regions was performed by Fab (meaning that a molecular form is Fab, the same applies below) phage display. The improvement of the complementarity determining regions was consisted of two steps: in the first step, one amino acid substitution aiming at improving the affinity to human PAD4 was determined, and in the second step, multiple combinations of these one amino acid substitutions were determined (Fujino et al., Biochem. Biophys. Res. Commun., 2012 Vol. 428 (3), p395). A Fab phage display vector was constructed using light chain and heavy chain variable regions of a parent clone G8. By multi-step PCR reactions involving a site-directed mutagenesis PCR and an overlap extension PCR using this as a template, a comprehensive 1-amino acid substitution mutant library in which all amino acid residues forming the 6 complementarity determining regions (LCDR1, LCDR2, LCDR3, HCDR1, HCDR2, HCDR3) of the antibody were substituted with 20 kinds of natural amino acids one by one. Using a Fab phage display method (Fujino et al., Biochem. Biophys. Res. Commun., 2012 Vol. 428 (3), p395), concentration of the comprehensive 1-amino acid substitution mutant library was repeated several rounds using the recombinant PAD4 protein (biotin-labeled PAD4) as a bait. Base sequences of light chain and heavy chain variable regions of clones contained in the library before the concentration (immediately after the construction) and the library after the concentration were analyzed using a next-generation sequencer (Ion GeneStudio S5 System). First, sequence data of millions of reads were obtained from the libraries before and after the concentration, and an existence frequency of all 1-amino acid substitution mutants in the complementarity determining regions was calculated. Next, a change magnification (concentration ratio) of the existence frequency of all 1-amino acid substitution mutants in the library before the concentration and the library after the concentration was calculated, and, using a magnitude of the concentration ratio as an index, a 1-amino acid substitution considered to be useful for improving the affinity to human PAD4 protein was determined. In this case, data of 1-amino acid substitutes (lysine and arginine) that may affect physical properties of the antibody were excluded. Finally, considering a total number of these 1-amino acid substitutions and the distribution on an amino acid sequence, positions where amino acid substitutions are to be introduced were for construction of second step custom library.

In the parent clone G8, it was determined to introduce amino acid substitutions into the second asparagine of LCDR2 (SEQ ID NO: 5 in the sequence listing), the fourth aspartic acid of LCDR3 (SEQ ID NO: 25 in the sequence listing), the 11th tyrosine, the 12th glycine, the 13th alanine, the 14th alanine, the 15th valine, and the 17th glycine of HCDR2 (SEQ ID NO: 24 in the sequence listing), and the first alanine of HCDR3 (SEQ ID NO: 11 of the sequence listing).

<Example 3> Creation of humanized anti-human PAD4 antibody with improved complementarity determining region

First, a full-fledged custom library for improving complementarity determining regions was designed by combining the above multiple useful amino acid substitutions for the purpose of affinity improvement. Next, a vector for parent clone G8 Fab phage display with a stop codon inserted into LCDR3 and HCDR2 was constructed, then a site-specific mutation introduction (Fellouse et al., J. Mol. Biol. 2007 Vol. 373, p924) by the Kunkel method was performed using this vector as a template, and thereby, a custom library for complementarity determining region improvement with complementarity determining regions randomized based on the above design was constructed. Using human PAD4 protein (His tag PAD4, GST-biotin-PAD4) as a bait, library concentration by Fab phage display was repeated for several rounds. Since a structure of PAD4 may change depending on presence or absence of calcium ions, selection was performed in presence and in absence of calcium chloride.

A recombinant protein used as a bait was prepared as follows. For human PAD4 (residues 1 to 663), an expression vector was constructed by inserting that obtained by adding GST tag-Avi tag to an N-terminal side into pGEX-6P-1, and a recombinant protein was prepared. An *Escherichia coli* BL21 (DE3) pLysS strain carrying the expression vector was precultured in 5 mL of an LB medium and then 1 mL of the preculture solution was inoculated into 50 mL of an LB medium, and was incubated at 37 °C until a logarithmic growth phase (OD600 = 1.0), and then, expression culture was performed with 100 μM IPTG at (18 °C)/(200 rpm) for about 18 hours. Collected bacterial cells were washed and then were crushed with ultrasonic waves and were subjected to bacteriolysis, and a supernatant was collected. A commercially available biotin ligase (Avidity, BirA) was caused to react with the supernatant at 4 °C for about 18 hours, and a supernatant was collected by centrifugal separation. GST tag-Avi tag-human PAD4 contained in the supernatant was purified using a GS4B resin (GE Healthcare), the GST tag was cleaved using PreScission Protease (GE Healthcare), and the Avi tag-human PAD4 was collected.

Clones contained in the Fab library concentrated in 4 - 6 rounds in presence and in absence of calcium chloride were cloned, and sequences of 384 clones randomly selected from each condition were identified using a sequencer. DNA fragments for expression were prepared in which 384 Fab clones were all converted into full-length antibodies (human IgGl/human λ), and the above DNA was introduced into Expi293 (Life Technologies) using gene transfer reagent ExpiFectamine 293 Transfection Kits (Life Technologies). After culturing for 5 days after gene transfer, a culture supernatant was obtained. A test antibody (IgG) (meaning that a molecular form is IgG, the same applies below) was purified from the culture supernatant by affinity chromatography using a Protein A resin (GE Healthcare, PreDictor MabSelect SuRe LX). The purified test antibody solution was subjected to solution substitution with PBS pH 7.4 using a dialysis device (Merck, D-Tube96 Dialyzer).

The test antibody was subjected to antigen binding confirmation by surface plasmon resonance (SPR). In the SPR experiment, the antigen binding of the test antibody in a running buffer solution HBS-EP+ (10 mM HEPES, 150 mM NaCl, 3 mM EDTA, 0.05% (v/v) Surfactant P20, pH 7.4) was confirmed using a surface plasmon resonance apparatus Biacore T200 (GE Healthcare). A biotin-labeled human PAD4 protein was immobilized on a sensor chip using a Biotin Capture Kit (GE Healthcare). The PAD4 protein was prepared to 4 $\mu$g/mL using HBS-EP+ and used as a ligand solution. The ligand solution was added to a flow cell at a flow rate of 10 $\mu$L/min for 30 seconds, and then HBS-EP+ was added for 60 seconds. A flow cell to which no ligand solution was added was used as a reference cell. The test antibody was prepared to 10 nM using HBS-EP+ and used as an analyte solution. Further, a running buffer solution was used as a blank solution. The blank solution or the analyte solution was added at a flow rate of 30 $\mu$L/min for 120 seconds to obtain a sensorgram of a bound phase. Next, a running buffer solution was added for 180 seconds to obtain a sensorgram of a dissociated phase. A sensorgram of the reference cell is subtracted from a sensorgram of the flow cell to which the ligand was added when the blank solution or the analyte solution was added, and further, a sensorgram to which the blank solution was added was subtracted from the sensorgram to which the analyte solution was added, and the result was used for analysis. A binding signal and non-specific adsorption were confirmed using Biacore T200 Evaluation software v3.1 (GE Healthcare).

From clones with improved complementarity determining regions, clones with improved affinity to human PAD4 protein and less non-specific binding to a sensor chip and clones that advance G8 to high-order evaluation after Example 4 were determined as 5 clones (G8, Lib2(4R)-1-47, Libl(6R)-1-39, Libl(6R)-2-37, and Lib2(5R)-2-25). CDR sequences of the five clones are shown in Table 1. Table 2 shows binding affinities of the five clones to human PAD4. Human PAD4 enzyme inhibitory activities of the test antibodies (G8, Lib2(4R)-1-47, Libl(6R)-1-39, Libl(6R)-2-37, and Lib2(5R)-2-25) were evaluated. Human PAD4 (final concentration: 10 nM) and a 50 mM HEPES buffer solution (pH 7.6) containing a 1 mM DTT and a 150 mM NaCl were mixed such that final concentrations of the test antibodies were 600, 200, 66.67, 22.22, 7.41, 2.47, 0.82, 0.27, and 0.09 nM. After incubating at 37 °C for 1 hour, BAEE (benzoylarginine ethyl ester) was added with stirring, and CaCl$_2$ was further added and the mixture was well stirred (a final concentration of BAEE was 10 mM, and a final concentration of calcium ions was 10 mM). After this solution is incubated at 37 °C for 3 hours, citrulline residues of citrullinated BAEE were subjected to colorimetric quantification using a mixed solution containing 2,3-butandionomonooxime and thiosemicarbazide.

The test antibodies (G8, Lib2(4R)-1-47, Libl(6R)-1-39, Libl(6R)-2-37, and Lib2(5R)-2-25) inhibited the enzyme activity of human PAD4, and IC50 values were respectively 21.6, 9.6, 8.7, 13.4 and 8.3 nM.

## Table 1

| | Light chain | | | | | | | | | | | | | | | | | | | | | | | | | |
|---|---|---|---|---|---|---|---|---|---|---|---|---|---|---|---|---|---|---|---|---|---|---|---|---|---|---|
| | CDR1 | | | | | | | | | | CDR2 | | | | | | | CDR3 | | | | | | | | |
| G8 | S | G | G | N | R | N | Y | Y | Y | G | A | N | D | K | R | P | S | G | T | A | D | T | G | K | Y | V |
| Lib2(4R)-1-47 | S | G | G | N | R | N | Y | Y | Y | G | A | N | D | K | R | P | S | G | T | A | T | T | G | K | Y | V |
| Lib1(6R)-1-39 | S | G | G | N | R | N | Y | Y | Y | G | A | N | D | K | R | P | S | G | T | A | T | T | G | K | Y | V |
| Lib1(6R)-2-37 | S | G | G | N | R | N | Y | Y | Y | G | A | N | D | K | R | P | S | G | T | A | L | T | G | K | Y | V |
| Lib2(5R)-2-25 | S | G | G | N | R | N | Y | Y | Y | G | A | N | D | K | R | P | S | G | T | A | Y | T | G | K | Y | V |

| | Heavy chain | | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|
| | CDR1 | | | | | | CDR2 | | | | | | | | | | | | | | | | CDR3 | | | | | | | | | | | | |
| G8 | T | Y | A | M | G | A | I | R | N | D | G | S | W | T | G | Y | G | A | A | V | K | G | A | K | Y | T | G | S | S | G | G | S | I | G | A |
| Lib2(4R)-1-47 | T | Y | A | M | G | A | I | R | N | D | G | S | W | T | G | Y | G | T | P | Y | K | T | A | K | Y | T | G | S | S | G | G | S | I | G | A |
| Lib1(6R)-1-39 | T | Y | A | M | G | A | I | R | N | D | G | S | W | T | G | T | S | V | N | A | K | T | A | K | Y | T | G | S | S | G | G | S | I | G | A |
| Lib1(6R)-2-37 | T | Y | A | M | G | A | I | R | N | D | G | S | W | T | G | I | G | Y | N | Q | K | S | G | K | Y | T | G | S | S | G | G | S | I | G | A |
| Lib2(5R)-2-25 | T | Y | A | M | G | A | I | R | N | D | G | S | W | T | G | Y | P | P | P | L | K | G | A | K | Y | T | G | S | S | G | G | S | I | G | A |

Table 2

| Clone | $k_{on}$ (1/Ms) | $k_{off}$ (1/s) | $kD$ (pM) |
|---|---|---|---|
| G8 | $7.03 \times 10^6$ | $1.46 \times 10^{-3}$ | 208 |
| Lib2 (4R)-1-47 | $9.39 \times 10^6$ | $1.64 \times 10^{-4}$ | 17.5 |
| Lib1 (6R) -1-39 | $5.90 \times 10^6$ | $1.08 \times 10^{-4}$ | 18.3 |
| Lib1 (6R)-2-31 | $10.1 \times 10^6$ | $3.61 \times 10^{-4}$ | 35.8 |
| Lib2 (5R) -2-25 | $12.8 \times 10^6$ | $2.02 \times 10^{-4}$ | 15.8 |

For the five clones (G8, Lib2(4R)-1-47, Libl(6R)-1-39, Libl(6R)-2-37, and Lib2(5R)-2-25), the 84th asparagine was substituted with serine, and G8ss, 2-1-47, 3-1-39, 3-2-37, and 4-2-25 were respectively obtained. Expression vectors for mammalian cells expressing IgG were constructed by inserting DNA encoding amino acid sequences of light chains and heavy chains of the test antibodies (G8ss, 2-1-47, 3-1-39, 3-2-37, and 4-2-25) on a downstream side of a CMV promoter. DNA sequences of the light chains of the clones respectively used the DNA sequences encoding SEQ ID NOs: 27, 17, 19, 21 and 23 in the sequence listing, and DNA sequences of the heavy chains respectively used the DNA sequences encoding SEQ ID NOs: 11 and 12 in the sequence listing. The above expression vector was introduced into ExpiCHO (Life Technologies) using a gene transfer reagent ExpiFectamine CHO Transfection Kit (Life Technologies). After culturing for 14 days after gene transfer, a culture supernatant was obtained. IgG was purified from the culture supernatant using AKTA pure 25M (GE Healthcare) and by affinity chromatography using a Protein A resin (JSR Life Sciences, Amsphere A3 column). IgG bound to the Protein A resin was washed with a 100 mM sodium carbonate buffer solution (pH 11.0) of 6 column volume and then was washed with PBS (pH 7.2) (3×) of 6 column volume. Then, it was eluted with a Gly-HCl buffer solution of pH 3.5 and neutralized promptly to bring the pH to near neutral. For a purpose of increasing purification purity, IgG after the protein A column purification was purified with CHT (ceramic hydroxyapatite Type I resin) (Bio-rad). IgG bound to CHT was eluted with a gradient of a NaCl concentration, a desired fraction was collected, and then solution substitution with PBS (pH 7.4) was performed using a dialysis method. Peptide mapping was performed on samples stored at 4 °C after purification of the test antibodies (G8ss, 2-1-47, 3-1-39, 3-2-37, and 4-2-25). As a result, no peptide cleaved between the 84th serine residue and the 85th serine residue was detected.

<Example 4> Binding affinity with respect to human PAD4 protein

Binding affinities of the test antibodies (G8ss, 2-1-47, 3-1-39, 3-2-37, and 4-2-25) with respect to human PAD4 proteins were determined by measuring a dissociation constant ($KD$) in HBS-EP+ using the surface plasmon resonance apparatus Biacore T200 (GE Healthcare). A biotin-labeled human PAD4 protein was immobilized on a sensor chip using a Biotin Capture Kit (GE Healthcare). The PAD4 protein was prepared to 0.5 μg/mL using HBS-EP+ and used as a ligand solution. The ligand solution was added to a flow cell at a flow rate of 10 μL/min for 30 seconds, and then HBS-EP+ was added for 60 seconds. A flow cell to which no ligand solution was added was used as a reference cell. The test antibody was prepared from several hundred pM to several nM using HBS-EP+ and used as an analyte solution. Further, a running buffer solution was used as a blank solution. The blank solution or the analyte solution was added at a flow rate of 30 μL/min for 180 seconds using a single-cycle method, and a dissociation time was 1200 seconds. A sensorgram of the reference cell is subtracted from a sensorgram of the flow cell to which the ligand was added when the blank solution or the analyte solution was added, and further, a sensorgram to which the blank solution was added was subtracted from the sensorgram to which the analyte solution was added, and the result was used for analysis. Binding parameters were calculated using a 1:1 binding model using Biacore T200 Evaluation software (GE Healthcare). The calculated binding parameters are shown in Table 3. As shown in Table 3, the test antibody G8ss bound to a human PAD4 protein with a binding affinity having a binding rate constant $k_{on}$ = $6.15 \times 10^6$ (1/Ms), a dissociation rate constant $k_{off}$ = $1.22 \times 10^{-3}$ (1/s), and a dissociation constant $KD$ = 199 pM. The dissociation constants of the antibodies 2-1-47, 3-1-39, 3-2-37, and 4-2-25 with improved complementarity determining regions with respect to human PAD4 protein were respectively 31, 48, 73, and 20 pM.

Table 3

| Clone | $k_{on}$ (1 IMs) | $k_{off}$ (1/s) | $KD$ (pM) |
|---|---|---|---|
| G8ss | $6.15 \times 10^6$ | $1.22 \times 10^{-3}$ | 199 |
| 2-1-47 | $1.61 \times 10^{'}$ | $5.07 \times 10^{-4}$ | 31 |

(continued)

| Clone | $k_{on}$ (1 IMs) | $k_{off}$ (1/s) | KD (pM) |
|---|---|---|---|
| 3-1-39 | $1.30 \times 10^7$ | $6.26 \times 10^{-4}$ | as |
| 3-2-37 | $8.51 \times 10^6$ | $6.17 \times 10^{-4}$ | 73 |
| 4-2-25 | $1.47 \times 10^7$ | $2.99 \times 10^{-4}$ | 20 |

<Example 5> Evaluation of storage stability of antibody

Retention of the antigen-binding activity is included as one of the indicators of the antibody storage stability. It is commonly known that the storage stability varies greatly depending on the antibody. For example, in the teaching of DiCara et al., mAbs, 2018 Vol. 10 (7), p1073, antigen-binding abilities of antibodies stored at 4 °C or 40 °C were compared. However, a relative antigen-binding activity of an antibody stored at 40 °C ranges from 30% or less to 100% of a relative antigen-binding activity of an antibody stored at 4 °C.
In order to evaluate the storage stabilities of the test antibodies (G8ss, 2-1-47, 3-1-39, 3-2-37, and 4-2-25), the test antibodies were each dissolved in a citric acid buffer solution (50 mM citric acid, 150 mM NaCl, pH 6.3) at a concentration of about 10 mg/mL and stored at 4 °C or 40 °C for 4 weeks.

In order to investigate an antigen-binding ability after the storage, antigen-binding activity measurements were performed using a surface plasmon resonance apparatus Biacore T200 (GE Healthcare). Cryopreserved test antibodies and test antibodies stored at 4 °C or 40 °C for 4 weeks were prepared to 10 μg/mL using a running buffer solution HBS-EP+. Using a Series S Sensorchip Protein A (GE Healthcare), a test antibody solution was added to a flow cell 2 at a flow rate of 10 μL/min for 60 seconds and immobilized on a sensor chip. A flow cell 1 was used as a reference cell. A binding amount 55 seconds after the completion of the addition of the test antibody to the flow cell 2 was subtracted from a binding amount of the flow cell 1 to obtain an antibody binding amount. Next, human PAD4 (Cayman Chemical company, Cat 10500) was prepared to 50 nM using HBS-EP+ and used as an analyte solution. The analyte solution was added at a flow rate of 30 μL/min for 120 seconds to obtain a sensorgram of a bound phase. A binding amount 5 seconds before the completion of the addition of the analyte solution to the flow cell 2 was subtracted from a binding amount of the flow cell 1 to obtain an antigen binding amount. Subsequently, a running buffer solution was added for 180 seconds to obtain a sensorgram of a dissociated phase. The measurements were performed at 25 °C. The antibody binding amount and the antigen binding amount were calculated using a data analysis program (GE Healthcare, Biacore T200 Evaluation Software v3.1), and the antigen binding amount under each condition was divided by the antibody binding amount to obtain a relative antigen binding amount. There was no change between the relative antigen-binding amount of a cryopreserved test antibody and the relative antigen-binding amount of a test antibody stored at 4 °C. The relative antigen-binding amounts of various test antibodies stored at 40 °C for 4 weeks were compared to the relative antigen-binding amounts of the test antibodies stored at 4 °C for 4 weeks, and the antigen-binding activities were determined.

As shown in Table 4, the antigen-binding activity of G8ss after storage at 40 °C for 4 weeks was 82.2%. 2-1-47, 3-1-39, 3-2-37, and 4-2-25 each maintained an antigen-binding activity of 90% or more after storage at 40 °C for 4 weeks. For example, as in the teaching of Pisupati et al., mAbs, 2017 Vol. 9 (7), p1197, an antibody drug Remicade (registered trademark) maintained an antigen-binding activity of 80% or more even after storage at 40 °C for 1 month. Further, as for stability of a biological activity of an antibody, it is desirable to maintain a biological activity of 80% or more, preferably 90% or more, as compared to a biological activity of the antibody at the time of preparing an antibody preparation (for example, see WO 2003/018056). From the above, good storage stabilities were observed for the test antibodies G8ss, 2-1-47, 3-1-39, 3-2-37, and 4-2-25.

Table 4

| Clone | Stored at 40°C·1 M |
|---|---|
| G8ss | 82.2 |
| 2-1-47 | 95.6 |
| 3-1-39 | 93.9 |
| 3-2-37 | 94.7 |
| 4-2-25 | 97.0 |

<Example 6> In vitro evaluation of human PAD4 enzyme inhibitory activity

Human PAD4 enzyme inhibitory activities of the test antibodies (G8ss, 3-2-37, 3-1-39, 4-2-25, and 2-1-47) were evaluated. Human PAD4 (final concentration: 10 nM) and a 50 mM HEPES buffer solution (pH 7.4) containing a 1 mM EDTA, a 1 mM DTT and a 150 mM NaCl were mixed such that final concentrations of the test antibodies were 600, 200, 66.67, 22.22, 7.41, 2.47, 0.82, 0.27, and 0.09 nM. After incubating at 37 °C for 1 hour, BAEE (benzoylarginine ethyl ester) was added with stirring, and $CaCl_2$ was further added and the mixture was well stirred (a final concentration of BAEE was 10 mM, and a final concentration of calcium ions was 10 mM). After this solution is incubated at 37 °C for 3 hours, citrulline residues of citrullinated BAEE were subjected to colorimetric quantification using a mixed solution containing 2,3-butandionomonooxime and thiosemicarbazide.

The test antibodies (G8ss, 3-2-37, 3-1-39, 4-2-25, and 2-1-47) inhibited the enzymatic activity of human PAD4, and IC50 values were respectively 20.3, 15.3, 8.8, 7.6 and 12.2 nM.

<Example 7> CAIA model

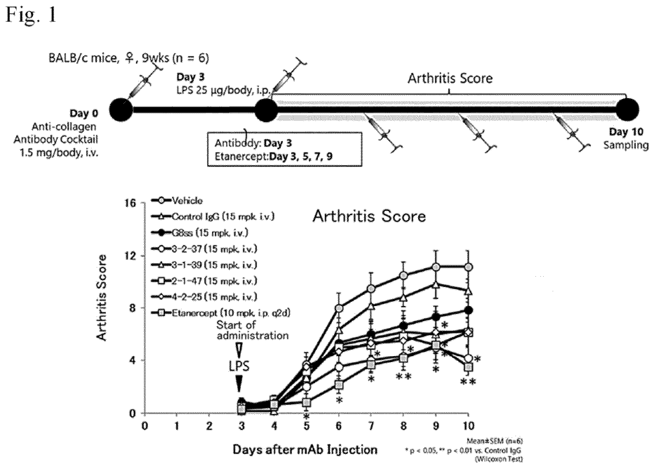

The efficacy of the test antibodies G8 ss, 2 -1 -47, 3 -1 -39, 3 -2 -37, and 4 -2 -25 was evaluated in anti-collagen antibody-induced arthritis (CAIA) model mice. The CAIA model mice are model mice of rheumatoid arthritis (RA) and arthritis. A method for producing the CAIA model mice followed a protocol of an antibody cocktail for inducing mouse arthritis (Chondrex Inc., Cat. 53040). Fig. 1 shows an outline of experimental conditions. On day 0, an anti-collagen antibody cocktail solution (1.5 mg) was intravenously administered to the tail vein of each of 9-week-old female Balb/c mice (6 mice/group). On day 3, 25 μg LPS (a substance that induces inflammation) was intraperitoneally administered. A test antibody or a control antibody (human IgG1) was intravenously administered (15 mg/kg) 4 hours before the LPS administration on day 3. On days 3 - 10, an arthritis score was evaluated according to (i) - (iii) below.

(i) The evaluation sites were each finger, insteps, and joints of the limbs.

(ii) Arthritis scores were assigned according to Table 5.

(iii) An arthritis score was indicated by an the mean of the sum of the score of each finger, insteps, and joints of the limbs (a maximum value is 16/mouse).

The evaluation results of the arthritis score are shown in Figure. As can be seen from this result, all the test antibodies had a high RA therapeutic effect.

Table 5

| Score | Symptoms |
|---|---|
| 0 | No inflammation is found in any joint |
| 1 | Inflammation is found in any one of the joints |
| 2 | Inflammation is found in any two of the joints |
| 3 | Inflammation is found in all the joints |
| 4 | Inflammation is found in all the joints and the entire limb swells red |

<Example 8> CIA model

The efficacy of test antibody 3 -2 -37 was evaluated in collagen-induced arthritis (CIA) model mice. 5-7 week old female DBA/1 mice were immunized with bovine type II collagen and Freund's adjuvant twice, on the first day and 21 days after the first immunization. Twentynine days after the first immunization, mice with arthritis were administered with the test antibody intravenously in the tail vein at a dose of 0.3 mg/kg, 1 mg/kg, or 3 mg/kg (9 mice per group). The same dose of 3-2-37 was administered 36 and 43 days after the first immunization. Arthritis scores were evaluated according to the following (i)-(iii).

(i) The evaluation sites were each finger, insteps, and joints of the limbs.

(ii) Arthritis scores were assigned according to Table 6.

(iii) An arthritis score was indicated by the mean of the sum of the scores of each finger, insteps, and joints of the limbs (a maximum value is 16/mouse)..

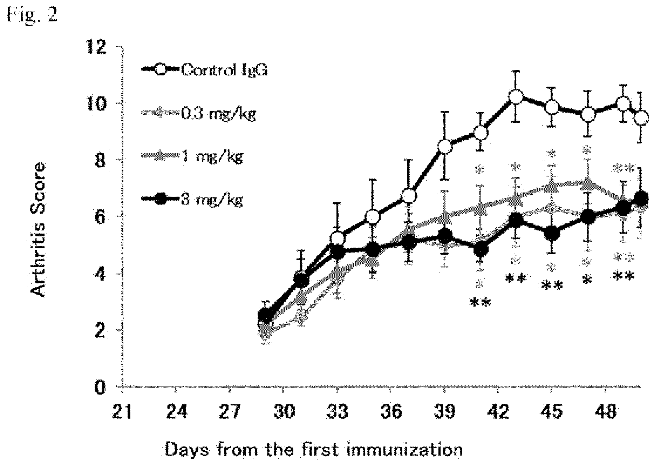

The evaluation results of the arthritis score are shown in Figure 2. As can be seen from the results, 3-2-37 significantly reduced the mean arthritis score against the control antibody group in the later half of the study at all doses set in this study (Shirley-Williams multiple comparison test, *p<0.025, **p<0.005).

Table 6

| Score | Symptoms |
|---|---|
| 0 | No inflammation is found in any joint |
| 1 | Inflammation is found in any one of the joints |
| 2 | Inflammation is found in any two of the joints |
| 3 | Severe inflammation of the instep and inflammation in each finger joint were found. |
| 4 | Severe inflammation and joint deformity of the entire limb with decreased range of motion was observed. |

<Example 9> cGvHD model

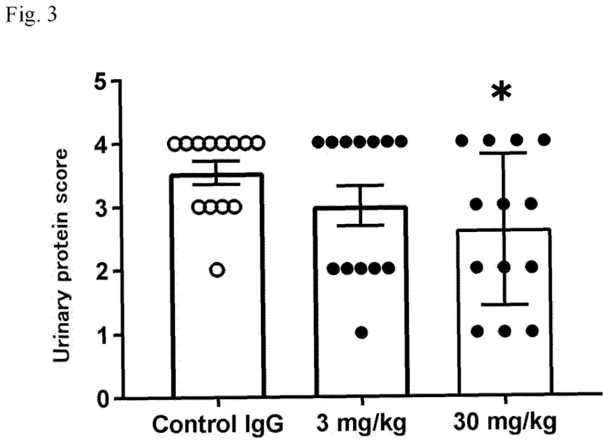

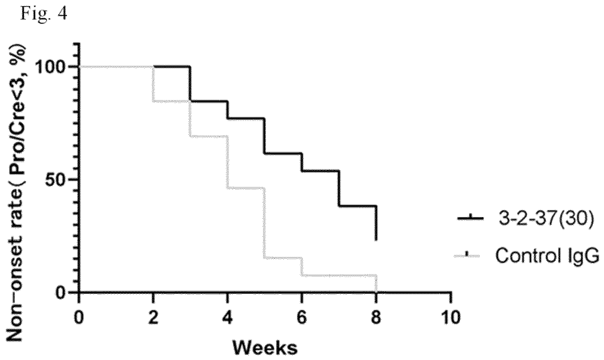

Chronic graft-versus-host disease (cGvHD) model mice were used to evaluate the efficacy of the test antibody 3-2-37. cGvHD model mice were generated by transferring spleen cells from 8-9 week old female DBA/2 mice into 8 week old female B6D2F1 mice. These mice develop lupus nephritis-like renal damage that occurs in SLE patients. The test antibodies were administered intraperitoneally at doses of 3 mg/kg or 30 mg/kg on the day before, 2 weeks, 4 weeks, and 6 weeks after splenocyte transfer (13 mice per group). From 2 weeks to 8 weeks after splenocyte transfer, urine was collected by weekly pressing the lower abdomen of the mice to measure protein and creatinine concentrations in the urine. The urine protein concentrations at 8 weeks after splenocyte transfer were scored according to Table 7. The evaluation results of urine protein scores are shown in Figure 3. As can be seen from the results, a significant decrease in urine protein score was observed in the 30 mg/kg test antibody 3-2-37-administered group compared to the control antibody group (Shirley-Williams multiple comparison test, *p<0.025). In addition, the urinary protein/creatinine ratio of less than 3 (Pro/Cre<3) was defined as a non-onset individual, and the results of graphing the non-onset rate in the 30 mg/kg test antibody 3-2-37-administered group and the control antibody group over time are shown in Figure 4. The median duration of non-onset in the test antibody-treated group was 7 weeks, whereas the median duration of non-onset in the control antibody-treated group was 4 weeks, indicating a significant prolongation of the duration of non-onset in the test antibody-treated group (LogRank test, p<0.05).

Table 7

| Score | Urinary protein concentration (mg/mL) |
|---|---|
| 0 | <0.3 |
| 1 | $0.3 \leq x < 1$ |
| 2 | $1 \leq x < 3$ |
| 3 | $3 \leq x < 10$ |
| 4 | $10 \leq$ |

<Description of sequence listing>

SEQ ID NO: 1: Amino acid sequence of HCDR1 of anti-PAD4 antibody

SEQ ID NO: 2: Amino acid sequence of HCDR2 of anti-PAD4 antibody (mixed sequence)

SEQ ID NO: 3: Amino acid sequence of HCDR3 of anti-PAD4 antibody (mixed sequence)

SEQ ID NO: 4: Amino acid sequence of LCDR1 of anti-PAD4 antibody

SEQ ID NO: 5: Amino acid sequence of LCDR2 of anti-PAD4 antibody

SEQ ID NO: 6: Amino acid sequence of LCDR3 of anti-PAD4 antibody (mixed sequence)

SEQ ID NO: 7: Amino acid sequence of HCDR2 of anti-PAD4 antibody Lib2 (4R)-1-47, 2-1-47

SEQ ID NO: 8: Amino acid sequence of HCDR2 of anti-PAD4 antibody Lib1 (6R)-1-39, 3-1-39

SEQ ID NO: 9: Amino acid sequence of HCDR2 of anti-PAD4 antibody Lib1 (6R)-2-37, 3-2-37

SEQ ID NO: 10: Amino acid sequence of HCDR2 of anti-PAD4 antibody Lib2 (5R)-2-25, 4-2-25

SEQ ID NO: 11: Amino acid sequence of HCDR3 of anti-PAD4 antibody G8, G8ss, Lib2 (4R)-1-47, 2-1-47, Lib1 (6R)-1-39, 3-1-39, Lib2 (5R)-2-25, 4-2-25

SEQ ID NO: 12: Amino acid sequence of HCDR3 of anti-PAD4 antibody Lib1 (6R)-2-37, 3-2-37

SEQ ID NO: 13: Amino acid sequence of LCDR3 of anti-PAD4 antibody Lib2 (4R)-1-47, 2-1-47, Lib1 (6R)-1-39, 3-1-39

SEQ ID NO: 14: Amino acid sequence of LCDR3 of anti-PAD4 antibody Lib1 (6R)-2-37, 3-2-37

SEQ ID NO: 15: Amino acid sequence of LCDR3 of anti-PAD4 antibody Lib2 (5R)-2-25, 4-2-25

SEQ ID NO: 16: Amino acid sequence of a full length of a heavy chain of anti-PAD4 antibody 2-1-47

SEQ ID NO: 17: Amino acid sequence of a full length of a light chain of anti-PAD4 antibody Lib2 (4R)-1-47, 2-1-47

SEQ ID NO: 18: Amino acid sequence of a full length of a heavy chain of anti-PAD4 antibody 3-1-39

SEQ ID NO: 19: Amino acid sequence of a full length of a light chain of anti-PAD4 antibody Lib1 (6R)-1-39, 3-1-39

SEQ ID NO: 20: Amino acid sequence of a full length of a heavy chain of anti-PAD4 antibody 3-2-37

SEQ ID NO: 21: Amino acid sequence of a full length of a light chain of anti-PAD4 antibody Lib1 (6R)-2-37, 3-2-37

SEQ ID NO: 22: Amino acid sequence of a full length of a heavy chain of anti-PAD4 antibody 4-2-25

SEQ ID NO: 23: Amino acid sequence of a full length of a light chain of anti-PAD4 antibody Lib2 (5R) -2-25, 4-2-25

SEQ ID NO: 24: Amino acid sequence of HCDR2 of anti-PAD4 antibody G8, G8ss

SEQ ID NO: 25: Amino acid sequence of LCDR3 of anti-PAD4 antibody G8, G8ss

SEQ ID NO: 26: Amino acid sequence of a full length of a heavy chain of anti-PAD4 antibody G8

SEQ ID NO: 27: Amino acid sequence of a full length of a light chain of anti-PAD4 antibody G8, G8ss

SEQ ID NO: 28: Amino acid sequence of a full length of a heavy chain of anti-PAD4 antibody G8ss

SEQ ID NO: 29: Amino acid sequence of PAD4

SEQ ID NO: 30: Base sequence encoding a full length of a heavy chain of anti-PAD4 antibody G8

SEQ ID NO: 31: Base sequence encoding a full length of a light chain of anti-PAD4 antibody G8, G8ss

SEQ ID NO: 32: Base sequence encoding a full length of a heavy chain of anti-PAD4 antibody 2-1-47

SEQ ID NO: 33: Base sequence encoding a full length of a light chain of anti-PAD4 antibody 2-1-47

SEQ ID NO: 34: Base sequence encoding a full length of a heavy chain of anti-PAD4 antibody 3-1-39

SEQ ID NO: 35: Base sequence encoding a full length of a light chain of anti-PAD4 antibody 3-1-39

SEQ ID NO: 36: Base sequence encoding a full length of a heavy chain of anti-PAD4 antibody 3-2-37

SEQ ID NO: 37: Base sequence encoding a full length of a light chain of anti-PAD4 antibody 3-2-37

SEQ ID NO: 38: Base sequence encoding a full length of a heavy chain of anti-PAD4 antibody 4-2-25

SEQ ID NO: 39: Base sequence encoding a full length of a light chain of anti-PAD4 antibody 4-2-25

SEQ ID NO: 40: Base sequence encoding a full length of a heavy chain of anti-PAD4 antibody G8ss

INDUSTRIAL APPLICABILITY

The present invention is useful for the prevention or treatment of RA or arthritis, systemic lupus erythematosus, lupus nephritis, or graft-versus-host disease, and thus has a high utility value in the pharmaceutical industry.

**Claims**

1. An anti-PAD4 antibody or an antibody fragment thereof, wherein HCDR1 comprises an amino acid sequence of SEQ ID NO: 1, HCDR2 comprises an amino acid sequence of SEQ ID NO: 2, HCDR3 comprises an amino acid sequence of SEQ ID NO: 3, LCDR1 comprises an amino acid sequence of SEQ ID NO: 4, LCDR2 comprises an amino acid sequence of SEQ ID NO: 5, and LCDR3 comprises an amino acid sequence of SEQ ID NO: 6.

2. The anti-PAD4 antibody or the antibody fragment thereof according to claim 1, wherein HCDR1 comprises the amino acid sequence of SEQ ID NO: 1, HCDR2 comprises any one of amino acid sequences of SEQ ID NOs: 7 - 10, HCDR3 comprises an amino acid sequence of SEQ ID NO: 11 or 12, LCDR1 comprises the amino acid sequence of SEQ ID NO: 4, LCDR2 comprises the amino acid sequence of SEQ ID NO: 5, and LCDR3 comprises any one of amino acid sequences of SEQ ID NOs: 13 -15.

3. The anti-PAD4 antibody or the antibody fragment thereof according to claim 1 or 2, wherein said anti-PAD4 antibody or the antibody fragment thereof is selected from the group consisting of:

   (a-1) an anti-PAD4 antibody or an antibody fragment thereof, wherein HCDR1 comprises the amino acid sequence of SEQ ID NO: 1, HCDR2 comprises the amino acid sequence of SEQ ID NO: 7, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 13;
   (b-1) an anti-PAD4 antibody or an antibody fragment thereof, wherein HCDR1 comprises the amino acid sequence of SEQ ID NO: 1, HCDR2 comprises the amino acid sequence of SEQ ID NO: 8, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 13;
   (c-1) an anti-PAD4 antibody or an antibody fragment thereof, wherein HCDR1 comprises the amino acid sequence of SEQ ID NO: 1, HCDR2 comprises the amino acid sequence of SEQ ID NO: 9, HCDR3 comprises the amino acid sequence of SEQ ID NO: 12, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 14; and
   (d-1) an anti-PAD4 antibody or an antibody fragment thereof, wherein HCDR1 comprises the amino acid sequence of SEQ ID NO: 1, HCDR2 comprises the amino acid sequence of SEQ ID NO: 10, HCDR3 comprises the amino acid sequence of SEQ ID NO: 11, LCDR1 comprises the amino acid sequence of SEQ ID NO: 4, LCDR2 comprises the amino acid sequence of SEQ ID NO: 5, and LCDR3 comprises the amino acid sequence of SEQ ID NO: 15.

4. The anti-PAD4 antibody or the antibody fragment thereof according to any one of claims 1 - 3, wherein said anti-PAD4 antibody or the antibody fragment thereof is selected from the group consisting of:

(a-2) an anti-PAD4 antibody or an antibody fragment thereof, wherein HCDR1 consists of the amino acid sequence of SEQ ID NO: 1, HCDR2 consists of the amino acid sequence of SEQ ID NO: 7, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 13;

(b-2) an anti-PAD4 antibody or an antibody fragment thereof, wherein HCDR1 consists of the amino acid sequence of SEQ ID NO: 1, HCDR2 consists of the amino acid sequence of SEQ ID NO: 8, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 13;

(c-2) an anti-PAD4 antibody or an antibody fragment thereof, wherein HCDR1 consists of the amino acid sequence of SEQ ID NO: 1, HCDR2 consists of the amino acid sequence of SEQ ID NO: 9, HCDR3 consists of the amino acid sequence of SEQ ID NO: 12, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 14; and

(d-2) an anti-PAD4 antibody or an antibody fragment thereof, wherein HCDR1 consists of the amino acid sequence of SEQ ID NO: 1, HCDR2 consists of the amino acid sequence of SEQ ID NO: 10, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 15.

5. An anti-PAD4 antibody or an antibody fragment thereof, wherein said anti-PAD4 antibody or the antibody fragment thereof is selected from the group consisting of:

(a-3) an anti-PAD4 antibody or an antibody fragment thereof, wherein a heavy chain variable region comprises an amino acid sequence of amino acid numbers 1 - 120 of SEQ ID NO: 16, and a light chain variable region comprises an amino acid sequence of amino acid numbers 1 - 105 of SEQ ID NO: 17;

(b-3) an anti-PAD4 antibody or an antibody fragment thereof, wherein a heavy chain variable region comprises an amino acid sequence of amino acid numbers 1 - 120 of SEQ ID NO: 18, and a light chain variable region comprises an amino acid sequence of amino acid numbers 1 - 105 of SEQ ID NO: 19;

(c-3) an anti-PAD4 antibody or an antibody fragment thereof, wherein a heavy chain variable region comprises an amino acid sequence of amino acid numbers 1 - 120 of SEQ ID NO: 20, and a light chain variable region comprises an amino acid sequence of amino acid numbers 1 - 105 of SEQ ID NO: 21; and

(d-3) an anti-PAD4 antibody or an antibody fragment thereof, wherein a heavy chain variable region comprises an amino acid sequence of amino acid numbers 1 - 120 of SEQ ID NO: 22, and a light chain variable region comprises an amino acid sequence of amino acid numbers 1 - 105 of SEQ ID NO: 23.

6. The anti-PAD4 antibody or the antibody fragment thereof according to claim 5, wherein said anti-PAD4 antibody or the antibody fragment thereof is selected from the group consisting of:

(a-4) an anti-PAD4 antibody or an antibody fragment thereof, wherein a heavy chain variable region consists of the amino acid sequence of amino acid numbers 1 - 120 of SEQ ID NO: 16, and a light chain variable region consists of the amino acid sequence of amino acid numbers 1 - 105 of SEQ ID NO: 17;

(b-4) an anti-PAD4 antibody or an antibody fragment thereof, wherein a heavy chain variable region consists of the amino acid sequence of amino acid numbers 1 - 120 of SEQ ID NO: 18, and a light chain variable region consists of the amino acid sequence of amino acid numbers 1 - 105 of SEQ ID NO: 19;

(c-4) an anti-PAD4 antibody or an antibody fragment thereof, wherein a heavy chain variable region consists of the amino acid sequence of amino acid numbers 1 - 120 of SEQ ID NO: 20, and a light chain variable region consists of the amino acid sequence of amino acid numbers 1 - 105 of SEQ ID NO: 21; and

(d-4) an anti-PAD4 antibody or an antibody fragment thereof, wherein a heavy chain variable region consists of the amino acid sequence of amino acid numbers 1 - 120 of SEQ ID NO: 22, and a light chain variable region consists of the amino acid sequence of amino acid numbers 1 - 105 of SEQ ID NO: 23.

7. The anti-PAD4 antibody or the antibody fragment thereof according to claim 5 or 6, wherein said anti-PAD4 antibody or the antibody fragment thereof is selected from the group consisting of:

(a-5) an anti-PAD4 antibody or an antibody fragment thereof, wherein a heavy chain consists of an amino acid sequence of SEQ ID NO: 16, and a light chain consists of an amino acid sequence of SEQ ID NO: 17;

(b-5) an anti-PAD4 antibody or an antibody fragment thereof, wherein a heavy chain consists of an amino acid sequence of SEQ ID NO: 18, and a light chain consists of an amino acid sequence of SEQ ID NO: 19;

(c-5) an anti-PAD4 antibody or an antibody fragment thereof, wherein a heavy chain consists of an amino acid sequence of SEQ ID NO: 20, and a light chain consists of an amino acid sequence of SEQ ID NO: 21; and

(d-5) an anti-PAD4 antibody or an antibody fragment thereof, wherein a heavy chain consists of an amino acid sequence of SEQ ID NO: 22, and a light chain consists of an amino acid sequence of SEQ ID NO: 23.

8. The anti-PAD4 antibody or the antibody fragment thereof according to any one of claims 1 - 7, wherein an amino acid corresponding to the 84th amino acid of a heavy chain is serine.

9. The anti-PAD4 antibody or the antibody fragment thereof according to any one of claims 1 - 8, wherein a KD value with respect to PAD4 is 100 pM or less.

10. The anti-PAD4 antibody or the antibody fragment thereof according to any one of claims 1 - 9, wherein said anti-PAD4 antibody or the antibody fragment thereof has a binding amount of 90% or more when the anti-PAD4 antibody or the antibody fragment thereof is stored at 40 °C for one month as compared to a PAD4-binding amount of the anti-PAD4 antibody or the antibody fragment thereof before the storage.

11. e-1) An anti-PAD4 antibody or an antibody fragment thereof, wherein HCDR1 comprises an amino acid sequence of SEQ ID NO: 1, HCDR2 comprises an amino acid sequence of SEQ ID NO: 24, HCDR3 comprises an amino acid sequence of SEQ ID NO: 11, LCDR1 comprises an amino acid sequence of SEQ ID NO: 4, LCDR2 comprises an amino acid sequence of SEQ ID NO: 5, and LCDR3 comprises an amino acid sequence of SEQ ID NO: 25, wherein an amino acid corresponding to the 84th amino acid of a heavy chain is serine.

12. e-2) The anti-PAD4 antibody or the antibody fragment thereof according to claim 11, wherein HCDR1 consists of the amino acid sequence of SEQ ID NO: 1, HCDR2 consists of the amino acid sequence of SEQ ID NO: 24, HCDR3 consists of the amino acid sequence of SEQ ID NO: 11, LCDR1 consists of the amino acid sequence of SEQ ID NO: 4, LCDR2 consists of the amino acid sequence of SEQ ID NO: 5, and LCDR3 consists of the amino acid sequence of SEQ ID NO: 25, wherein the amino acid corresponding to the 84th amino acid of a heavy chain is serine.

13. e-4) An anti-PAD4 antibody or an antibody fragment thereof, wherein a heavy chain variable region comprises an amino acid sequence of amino acid numbers 1 - 120 of SEQ ID NO: 28, and a light chain variable region comprises an amino acid sequence of amino acid numbers 1 - 105 of SEQ ID NO: 27.

14. e-5) The anti-PAD4 antibody or the antibody fragment thereof according to claim 13, wherein a heavy chain variable region consists of the amino acid sequence of amino acid numbers 1 - 120 of SEQ ID NO: 28, and a light chain variable region consists of the amino acid sequence of amino acid numbers 1 - 105 of SEQ ID NO: 27.

15. The anti-PAD4 antibody or the antibody fragment thereof according to claim 13 or 14, wherein a heavy chain consists of SEQ ID NO: 28 and a light chain consists of SEQ ID NO: 27.

16. The anti-PAD4 antibody or the antibody fragment thereof according to any one of claims 11 - 15, wherein cleavage occurring during generation is less than 3% of a total generation amount.

17. The anti-PAD4 antibody or the antibody fragment thereof according to any one of claims 1 - 16, wherein the antibody is a neutralizing antibody.

18. The anti-PAD4 antibody or the antibody fragment thereof according to any one of claims 1 - 17, wherein the antibody is a PAD4 binding antibody represented by SEQ ID NO: 29.

19. A nucleic acid molecule comprising a base sequence encoding the anti-PAD4 antibody or the antibody fragment thereof according to any one of claims 1-15.

20. A recombinant vector comprising the nucleic acid molecule according claim 19.

21. A transformant comprising the recombinant vector according to claim 20.

22. A pharmaceutical composition comprising the anti-PAD4 antibody or the antibody fragment thereof according to any one of claims 1-18.

23. The pharmaceutical composition according to claim 22, wherein said pharmaceutical composition is a preventive agent or a therapeutic agent for rheumatoid arthritis or arthritis, systemic lupus erythematosus, lupus nephritis, or graft-versus-host disease.

24. The pharmaceutical composition according to claim 22 or 23, wherein said pharmaceutical composition is an inhibiting agent of citrullination in a protein.

25. The pharmaceutical composition according to any one of claims 22 - 24, wherein said pharmaceutical composition is an inhibiting agent of netosis in cells.

26. Use of the anti-PAD4 antibody or the antibody fragment thereof according to any one of claims 1 - 18 or the pharmaceutical composition according to any one of claims 22 - 25 for preventing or treating rheumatoid arthritis or arthritis, systemic lupus erythematosus, lupus nephritis, or graft-versus-host disease.

27. Use of the anti-PAD4 antibody or the antibody fragment thereof according to any one of claims 1 - 18 or the pharmaceutical composition according to any one of claims 22 - 25 in manufacture of a preventive agent or a therapeutic agent for rheumatoid arthritis or arthritis, systemic lupus erythematosus, lupus nephritis, or graft-versus-host disease.

28. A method for preventing or treating rheumatoid arthritis or arthritis, systemic lupus erythematosus, lupus nephritis, or graft-versus-host disease, said method comprising administering an effective amount of the anti-PAD4 antibody or the antibody fragment thereof according to any one of claims 1 - 18 or the pharmaceutical composition according to any one of claims 22 - 25.

Fig. 1

Fig. 2

**Days from the first immunization**

Fig. 3

Fig. 4

## INTERNATIONAL SEARCH REPORT

| International application No. |
|---|
| **PCT/JP2022/006528** |

### A. CLASSIFICATION OF SUBJECT MATTER

*C12N 15/13*(2006.01)i; *A61K 39/395*(2006.01)i; *A61P 13/12*(2006.01)i; *A61P 19/02*(2006.01)i; *A61P 29/00*(2006.01)i; *A61P 37/02*(2006.01)i; *A61P 37/06*(2006.01)i; *A61P 43/00*(2006.01)i; *C07K 16/40*(2006.01)i; *C12N 1/15*(2006.01)i; *C12N 1/19*(2006.01)i; *C12N 1/21*(2006.01)i; *C12N 5/10*(2006.01)i

FI: C12N15/13; C12N1/15; C12N1/19; C12N1/21; C12N5/10; A61K39/395 D; A61K39/395 N; A61P19/02; A61P29/00 101; A61P37/02; A61P13/12; A61P37/06; A61P43/00 111; C07K16/40 ZNA

According to International Patent Classification (IPC) or to both national classification and IPC

### B. FIELDS SEARCHED

Minimum documentation searched (classification system followed by classification symbols)

C12N15/13; A61K39/395; A61P13/12; A61P19/02; A61P29/00; A61P37/02; A61P37/06; A61P43/00; C07K16/40; C12N1/15; C12N1/19; C12N1/21; C12N5/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq

### C. DOCUMENTS CONSIDERED TO BE RELEVANT

| Category* | Citation of document, with indication, where appropriate, of the relevant passages | Relevant to claim No. |
|---|---|---|
| Y | WO 2016/143753 A1 (PUBLIC UNIVERSITY CORP. YOKOHAMA CITY UNIVERSITY) 15 September 2016 (2016-09-15) claims, example 1 | 1-4, 8, 9, 11, 12, 17-28 |
| A | claims, example 1 | 5-7, 10, 13-16 |
| Y | JP 2011-507519 A (CENTOCOR ORTHO BIOTECH INC.) 10 March 2011 (2011-03-10) claims, paragraphs [0004], [0018] | 1-4, 8, 9, 11, 12, 17-28 |
| A | claims, paragraphs [0004], [0018] | 5-7, 10, 13-16 |
| Y | JP 2008-536490 A (NATIONAL RESEARCH COUNC. OF CANADA) 11 September 2008 (2008-09-11) claims, paragraph [0040] | 1-4, 8, 9, 11, 12, 17-28 |
| A | claims, paragraph [0040] | 5-7, 10, 13-16 |

☑ Further documents are listed in the continuation of Box C. ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **26 April 2022** | **17 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
|---|
| **PCT/JP2022/006528** |

**C.     DOCUMENTS CONSIDERED TO BE RELEVANT**

| Category* | Citation of document, with indication, where appropriate, of the relevant passages | Relevant to claim No. |
|---|---|---|
| Y | JP 2007-526756 A (GENENTECH, INC.) 20 September 2007 (2007-09-20) | 1-4, 8, 9, 11, 12, 17-28 |
| | claims, abstract, paragraph [0062] | |
| A | claims, abstract, paragraph [0062] | 5-7, 10, 13-16 |
| Y | FUJINO, Y. et al. Robust in vitro affinity maturation strategy based on interface-focused high-throughput mutational scanning. Biochemical and Biophysical Research Communications. 2012, vol. 428, pages 395-400 | 1-4, 8, 9, 11, 12, 17-28 |
| | particularly, page 395, abstract | |
| A | particularly, page 395, abstract | 5-7, 10, 13-16 |

Form PCT/ISA/210 (second sheet) (January 2015)

**INTERNATIONAL SEARCH REPORT**

International application No.

**PCT/JP2022/006528**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.   ☑  forming part of the international application as filed:

           ☑  in the form of an Annex C/ST.25 text file.

           ☐  on paper or in the form of an image file.

    b.   ☐  furnished together with the international application under PCT Rule 13*ter*.1(a) for the purposes of international search only in the form of an Annex C/ST.25 text file.

    c.   ☐  furnished subsequent to the international filing date for the purposes of international search only:

           ☐  in the form of an Annex C/ST.25 text file (Rule 13*ter*.1(a)).

           ☐  on paper or in the form of an image file (Rule 13*ter*.1(b) and Administrative Instructions, Section 713).

2.  ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

EP 4 296 357 A1

**INTERNATIONAL SEARCH REPORT**
Information on patent family members

| International application No. |
| --- |
| **PCT/JP2022/006528** |

| Patent document cited in search report | | | Publication date (day/month/year) | Patent family member(s) | | | Publication date (day/month/year) |
| --- | --- | --- | --- | --- | --- | --- | --- |
| WO | 2016/143753 | A1 | 15 September 2016 | US | 2018/0044434 | A1 | |
| | | | | claims, example 1 | | | |
| | | | | EP | 3266872 | A1 | |
| JP | 2011-507519 | A | 10 March 2011 | US | 2010/0021477 | A1 | |
| | | | | WO | 2009/085462 | A1 | |
| | | | | claims, page 1, line 33 to page 2, line 2, page 4, lines 24-32 | | | |
| JP | 2008-536490 | A | 11 September 2008 | JP | 2012-70748 | A | |
| | | | | JP | 2014-144010 | A | |
| | | | | JP | 2016-73284 | A | |
| | | | | WO | 2006/099747 | A1 | |
| | | | | claims, page 14, lines 15-26 | | | |
| | | | | JP | 2016-103998 | A | |
| | | | | JP | 2018-110583 | A | |
| | | | | JP | 2019-187439 | A | |
| | | | | US | 2009/0220485 | A1 | |
| | | | | US | 2013/0142780 | A1 | |
| | | | | US | 2016/0052995 | A1 | |
| | | | | US | 2019/0092840 | A1 | |
| | | | | EP | 2316945 | A1 | |
| | | | | EP | 2316946 | A1 | |
| | | | | EP | 2316947 | A1 | |
| | | | | EP | 2316948 | A1 | |
| JP | 2007-526756 | A | 20 September 2007 | JP | 2007-501011 | A | |
| | | | | WO | 2005/012359 | A2 | |
| | | | | claims, abstract, page 30, line 38 to page 31, line 12 | | | |
| | | | | JP | 2011-46732 | A | |
| | | | | US | 2005/0106667 | A1 | |
| | | | | US | 2006/0280747 | A1 | |
| | | | | US | 2007/0020267 | A1 | |
| | | | | US | 2007/0141065 | A1 | |
| | | | | WO | 2005/012531 | A2 | |
| | | | | WO | 2005/044853 | A2 | |
| | | | | EP | 2420512 | A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012026309 A
- WO 2016143753 A
- JP H4506458 B
- JP 2912618 B
- JP H4504365 B
- JP H7509137 B
- WO 9425585 A
- JP H6500233 B
- WO 2005035586 A
- WO 200231140 A
- WO 0061739 A
- US 6737056 B
- US 7297775 B
- US 7317091 B
- US 4737456 A
- WO 2016175236 A
- WO 2003018056 A

### Non-patent literature cited in the description

- **SUZUKI et al.** Functional haplotypes of PADI4, encoding citrullinating enzyme peptidylarginine deiminase 4, are associated with rheumatoid arthritis. *Nat Genet.,* August 2003, vol. 34 (4), 395-402
- **ISHIGAMI et al.** Two novel sandwich ELISAs identify PAD4 levels and PAD4 autoantibodies in patients with rheumatoid arthritis. *Mod Rheumatol.,* July 2013, vol. 23 (4), 794-803
- **YUDONG et al.** Peptidylarginine deiminases 2 and 4 modulate innate and adaptive immune responses in TLR-7-dependent lupus. *JCI Insight.,* 06 December 2018, vol. 3 (23), e124729
- **HANATA et al.** Peptidylarginine Deiminase 4 Promotes the Renal Infiltration of Neutrophils and Exacerbates the TLR7 Agonist-Induced Lupus Mice. *Front Immunol.,* June 2020, vol. 23 (11), 1095
- **KNIGHT et al.** Peptidylarginine deiminase inhibition disrupts NET formation and protects against kidney, skin and vascular disease in lupus-prone MRL/lpr mice. *Ann Rheum Dis.,* December 2015, vol. 74 (12), 2199-2206
- **GORDON et al.** Lupus and proliferative nephritis are PAD4 independent in murine models. *JCI Insight.,* 18 May 2017, vol. 2 (10), e92926
- **KABAT et al.** Sequences of Proteins of Immunological Interest. US Department of Health and Human Services, NIH, 1987
- Current Protocols in Molecular Biology. John Wiley & Sons, 1987
- Antibodies: A Laboratory Manual. Cold Spring Harbor Laboratory, 1988
- **KOEHLER ; MILSTEIN et al.** *Nature,* 1975, vol. 256 (495
- **ALMAGRO et al.** *FRont Biosci.,* 01 January 2008, vol. 13, 1619-1633
- **OZAKI et al.** *Blood,* 01 June 1999, vol. 93 (11), 3922-3930
- **ROGUSKA et al.** *Proc Natl Acad Sci USA,* 01 February 1994, vol. 91 (3), 969-973
- **DAMSCHRODER et al.** *Mol Immunol.,* 22 January 2007, vol. 44 (11), 3049-3060
- **RIECHMANN et al.** *Nature,* 24 March 1988, vol. 332 (6162), 323-327
- **NISHIBORI et al.** *Mol Immunol,* February 2006, vol. 43 (6), 634-42
- *Nature Genetics,* 1994, vol. 7, 13-21
- *Nature Genetics,* 1997, vol. 15, 146-156
- *Nature,* 1994, vol. 368, 856-859
- *Nucleic Acids Research,* 1986, vol. 14, 1779
- *The Journal of Biological Chemistry,* 1982, vol. 257, 1516
- *Cell,* 1980, vol. 22, 197
- **WU et al.** *Nature Biotechnology,* 2007, vol. 25 (11), 1290
- **KITAZAWA et al.** *Nature Medicine,* 2012, vol. 18 (10), 1570
- **KENYA SHITARA.** *Journal of Pharmacy,* 2009, vol. 129 (1), 3
- **AKIKO ISHII et al.** *Journal of Japanese Pharmacology,* 2010, vol. 136 (5), 280
- **SHUHEI HASHIGUCHI et al.** *Biochemistry,* 2010, vol. 82 (8), 710
- **SHUHEI HASHIGUCHI.** *Biochemistry,* 2010, vol. 82 (8), 710
- **D. J. KING.** Applications and Engineering of Monoclonal antibodies. T. J. International Ltd, 1998
- Monoclonal Antibody-Based Therapy of Cancer. Marcel Dekker Inc, 1998
- **CHARI et al.** *Cancer Res,* 1992, vol. 152, 127
- **LIU et al.** *Proc Natl Acad Sci USA.,* 1996, vol. 93, 8681

- **D.J.KING.** Applications and Engineering of Monoclonal antibodies. T.J.International Ltd, 1998
- **CHARI et al.** *Cancer Res.,* 1992, vol. 152, 127
- *Nucleic Acids Research,* 1986, vol. 14, 1779
- Antibodies: A Laboratory Manual. Cold Spring Harbor Laboratory, 1988
- **FUJINO et al.** *Biochem. Biophys. Res. Commun.,* 2012, vol. 428
- **FUJINO et al.** *Biochem. Biophys. Res. Commun.,* 2012, vol. 428 (3), 395
- **FELLOUSE et al.** *J. Mol. Biol.,* 2007, vol. 373, 924
- **DICARA et al.** *mAbs,* 2018, vol. 10
- **PISUPATI et al.** *mAbs,* 2017, vol. 9 (7), 1197